# EUROPEAN PATENT APPLICATION

(11) **EP 2 578 151 A1**
(43) Date of publication of application: **10.04.2013**
(21) Application number: 11789832.0
(22) Date of filing: 31.05.2011
(51) Int. Cl.: A61B 5/11

(54) **MOBILE TERMINAL DEVICE, WALKING LOCI CALCULATION PROGRAM AND WALKING POSTURE DIAGNOSIS METHOD**

(30) Priority: 02.06.2010 JP 2010127232
(71) Applicant: Fujitsu Limited, Kawasaki-shi, Kanagawa 211-8588 (JP)
(72) Inventor: KURIBAYASHI, Takamitsu, Kawasaki-shi Kanagawa 211-8588 (JP); YAMAGUCHI, Katsuyoshi, Kawasaki-shi Kanagawa 211-8588 (JP); YAMAMOTO, Tatsuya, Kato-shi Hyogo 673-1447 (JP); OMIYA, Rie, Kawasaki-shi Kanagawa 211-8588 (JP); NAGASHIMA, Fumio, Kawasaki-shi Kanagawa 211-8588 (JP); AKAMA, Katsuaki, Kawasaki-shi Kanagawa 211-8588 (JP); GOTO, Soichiro, Kato-shi Hyogo 673-1447 (JP); CHO, Hun Hong, Kato-shi Hyogo 673-1447 (JP); OONISHI, Yoshikazu, Kato-shi Hyogo 673-1447 (JP); NAKANO, Takahiro, Kato-shi Hyogo 673-1447 (JP); FUJII, Naoto, Kato-shi Hyogo 673-1447 (JP); MIURA, Atsushi, Kawasaki-shi Kanagawa 211-8588 (JP); YAMAMOTO, Yoshinori, Kawasaki-shi Kanagawa 211-8588 (JP); OSANAI, Iriichi, Kawasaki-shi Kanagawa 211-8588 (JP)
(74) Representative: Lewin, David Nicholas
(86) International application number: PCT/JP2011/062546
(87) International publication number: WO 2011/152429

(57) **Abstract**

A mobile terminal device (1) includes a first displacement detection unit (11) configured to detect a displacement value associated with movement in an up-and-down direction of the mobile terminal device, a second displacement detection unit (12) configured to detect a displacement value associated with movement or turning in a predetermined direction of the mobile terminal device, a walking posture reference locus storage unit (13) configured to store a locus serving as a reference in relation to a walking posture, a characteristic displacement timing extraction unit (14) configured to extract a timing of a predetermined characteristic displacement appearing at every step based on detected upward and downward displacement values at a plurality of timings, a locus calculation unit (15) configured to select extracted successive walking timings, and to calculate a locus caused by walking of the selected walking timings based on a displacement value detected by the second displacement detection unit, and a walking posture determination unit (16) configured to determine whether the calculated locus satisfies the reference in relation to the walking posture stored in the walking posture reference locus storage unit, whereby, a locus of a walking posture can be efficiently obtained.

## Description

### [Technical Field]

The present invention relates to a mobile electronic device, a walking locus calculation program, and a walking posture diagnostic method.

### [Background Art]

In recent years, a technology of defecting a position or a posture of an object to be measured such as a human body by combining an acceleration sensor and an angular velocity sensor has been proposed. For example, there is a technology in which information indicating the posture of the object to be measured is obtained by a six-axis sensor that includes both of a triaxial acceleration sensor that measures acceleration of the object to be measured and a triaxial angular velocity sensor that measures angular velocity of the object to be measured.

Also, there is a technology in which acceleration of a reference coordinate system in the six-axis sensor is calculated from each output data of the six-axis sensor based on a predetermined triaxial batch rotational transform technology and the position and the posture of the object to be measured are obtained even when the object to be measured turns around and the like.

### [Citation List]

### [Patent Citation]

Patent Literature 1: Japanese Laid-open Patent Publication No. 2010-32296
Patent Literature 2: International Publication Pamphlet No. WO 2008/026357
Patent Literature 3: Japanese Laid-open Patent Publication No. 2000-325329

### [Summary of Invention]

### [Technical Problem]

However, when the object to be measured is a portion of a human body, the conventional technology using the six-axis sensor has a problem that it is difficult to obtain a locus of a posture during walking. That is, the conventional technology can obtain information in relation to the position and posture of the portion of the body during walking, but it is difficult to obtain the locus of the posture during walking from each piece of output data of the six-axis sensor.

The technology to be disclosed has been made in view of the foregoing, and a purpose is to provide a mobile electronic device, a walking locus calculation program, and a walking posture diagnostic method that are capable of efficiently obtaining a locus of a walking posture even if an object to be measured is a portion of a human body. Advantageous Effects of Invention

### [Solution to Problem]

According to an aspect of an embodiment, a mobile electronic device includes a first displacement detection unit, a second displacement detection unit, a walking posture reference locus storage unit, a characteristic displacement timing extraction unit, a locus calculation unit and a walking posture determination unit. The first displacement detection unit detects a displacement value associated with movement in an up-and-down direction of the mobile electronic device. The second displacement detection unit detects a displacement value associated with movement or turning in a predetermined direction of the mobile electronic device. The walking posture reference locus storage unit stores a locus serving as a reference in relation to a walking posture. The characteristic displacement timing extraction unit extracts a timing of a predetermined characteristic displacement appearing at every step as a walking timing based on upward and downward displacement values detected at a plurality of timings by the first displacement detection unit. The locus calculation unit selects successive walking timings extracted by the characteristic displacement timing extraction unit, and calculates a locus caused by walking of the selected walking timings based on the displacement value detected by the second displacement detection unit. The walking posture determination unit determines whether the locus calculated by the locus calculation unit satisfies the reference in relation to a walking posture stored in the walking posture reference locus storage unit.

### [Advantageous Effects of Invention]

According to one aspect of the mobile electronic device disclosed in the present invention exhibits an effect of efficiently obtaining a locus of a walking posture.

### [Brief Description of Drawings]

FIG. 1 is a functional block diagram illustrating a configuration of a mobile terminal device according to an embodiment 1.
FIG. 2 is a functional block diagram illustrating a configuration of a mobile terminal device according to an embodiment 2.
FIG. 3 is a diagram describing a measurement amount of a six-axis sensor.
FIG. 4 is a diagram illustrating an example of a data structure of a diagnostic threshold value table.
FIG. 5 is a diagram illustrating an example of a data structure of a diagnostic advice table.
FIG. 6 is a diagram illustrating an example of data (raw data) output from the six-axis sensor.
FIG. 7 is a diagram illustrating an example of data temporarily used in a step marker extraction process.
FIG. 8 is a diagram illustrating an example of data output in the step marker extraction process.
FIG. 9 is a diagram illustrating an example of data output in a step marker right-and-left determination process.
FIG. 10 is a diagram illustrating an example of calculation data (no score and no form) of various moving loci using a result of a walking posture locus amount calculation process.
FIG. 11 is a diagram illustrating an example of calculation data (with a score and a form) of various moving loci using the result of the walking posture locus amount calculation process.
FIG. 12A is an explanation diagram describing a specific example of step marker extraction.
FIG. 12B is an explanation diagram describing a specific example of the step marker extraction.
FIG. 12C is an explanation diagram describing a specific example of the step marker extraction.
FIG. 13 is an explanation diagram describing a specific example of step marker right-and-left determination.
FIG. 14 is an explanation diagram describing a specific example of front-back turning amount calculation.
FIG. 15 is an explanation diagram describing a specific example of trunk turning amount calculation.
FIG. 16 is an explanation diagram describing a specific example of right-and-left turning amount calculation.
FIG. 17 is an explanation diagram describing a specific example of right-and-left moving distance calculation.
FIG. 18 is an explanation diagram describing a specific example of up-and-down moving distance calculation.
FIG. 19 is an explanation diagram describing a specific example of landing impact calculation.
FIG. 20A is a flowchart illustrating a procedure of the step marker extraction process according to the embodiment 2.
FIG. 20B is a flowchart illustrating a procedure of the step marker right-and-left determination process according to the embodiment 2.
FIG. 20C is a flowchart illustrating a procedure of the walking posture locus amount calculation process according to the embodiment 2.
FIG. 20D is a flowchart illustrating a procedure of the walking posture diagnostic process according to the embodiment 2.
FIG. 21 is a diagram illustrating an example of an equipped position of the six-axis sensor when the mobile terminal device is a mobile phone.
FIG. 22A is a diagram illustrating an example of a display screen.
FIG. 22B is a diagram illustrating an example of a display screen.
FIG. 22C is a diagram illustrating an example of a display screen.
FIG. 22D is a diagram illustrating an example of a display screen.
FIG. 22E is a diagram illustrating an example of a display screen.
FIG. 22F is a diagram illustrating an example of a display screen.
FIG. 22G is a diagram illustrating an example of a display screen.
FIG. 23 is a diagram illustrating a computer that executes a walking locus calculation program.

### [Embodiment for Carrying Out the Invention]

Hereinafter, embodiments of a mobile electronic device, a walking locus calculation program, and a walking posture diagnostic method disclosed by the present invention will be described in detail with reference to the drawings. Note that this invention is not limited according to the present embodiments.

### Embodiment 1

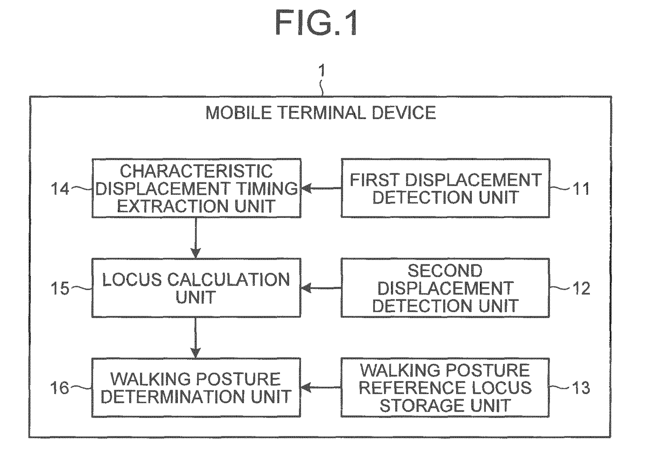

FIG. 1 is a functional block diagram illustrating a configuration of a mobile terminal device according to the present embodiment 1. As illustrated in FIG. 1, a mobile terminal device 1 includes a first displacement detection unit 11, a second displacement detection unit 12, a walking posture reference locus storage unit 13, a characteristic displacement timing extraction unit 14, a locus calculation unit 15, and a walking posture determination unit 16.

The first displacement detection unit 11 detects a displacement value associated with a movement in an up-and-down direction in walking of a subject. The second displacement detection unit 12 detects a displacement value associated with a movement or turning in a predetermined direction in walking of the subject. The walking posture reference locus storage unit 13 stores a locus that serves as a reference in relation to a walking posture.

The characteristic displacement timing extraction unit 14 extracts a timing of a predetermined characteristic displacement that appears at every step based on upward and downward displacement values at a plurality of timings detected by the first displacement detection unit 11. The locus calculation unit 15 selects successive walking timings extracted by the characteristic displacement timing extraction unit 14, and calculates a locus caused by the walking of the selected walking timings based on the displacement value detected by the second displacement detection unit 12.

The walking posture determination unit 16 determines whether the locus calculated by the locus calculation unit 15 satisfies the reference in relation to a walking posture stored in the walking posture reference locus storage unit 13.

In this way, the mobile terminal device 1 extracts a timing of a predetermined characteristic displacement that appears at every step as the walking timing based on upward and downward displacement values in walking of the subject. Therefore, the mobile terminal device 1 can obtain a displacement value associated with the movement or the turning in relation to a step of the extracted walking timing. Therefore, the mobile terminal device 1 can obtain a displacement value in relation to each step of the successive walking timings because of walking, and can efficiently calculate a locus of a walking posture caused by the walking based on each of the obtained displacement values. Further, the mobile terminal device 1 can efficiently diagnose the walking posture of the subject by using a locus serving as a reference in relation to the walking posture for comparison with the locus of the calculated walking posture.

### Embodiment 2

### [A configuration of a mobile terminal device according to an embodiment 2]

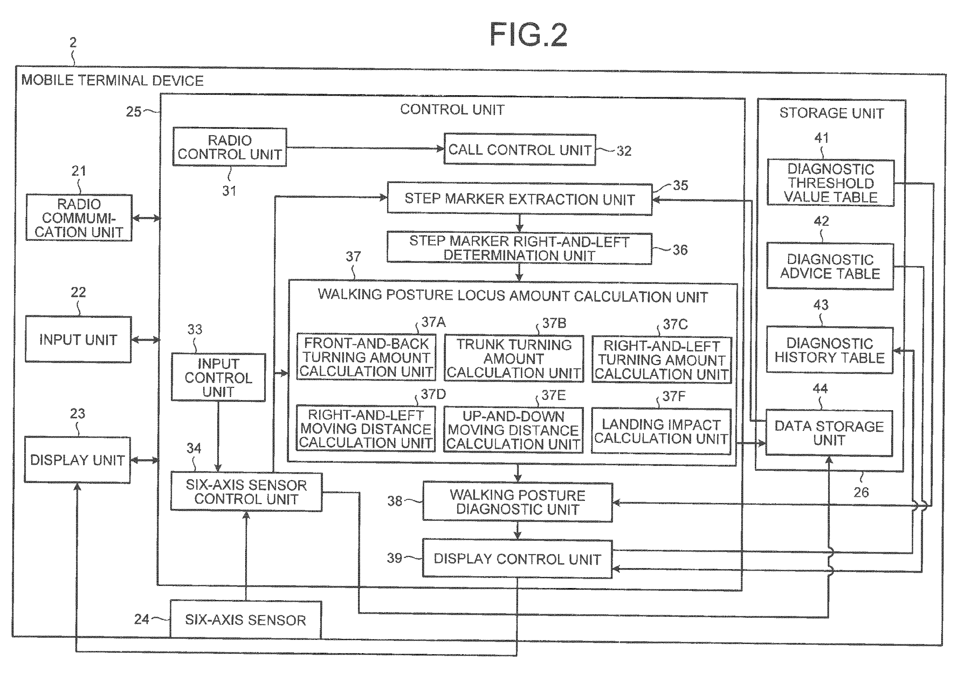

FIG. 2 is a functional block diagram illustrating a configuration of a mobile terminal device 2 according to the present embodiment 2. As illustrated in FIG. 2, the mobile terminal device 2 includes a radio communication unit 21, an input unit 22, a display unit 23, a six-axis sensor 24, a control unit 25, and a storage unit 26. Note that the mobile terminal device 2 is described as a mobile phone in the embodiment 2. However, the mobile terminal device 2 is not limited to the mobile phone as long as it is a movable terminal device.

The control unit 25 includes a radio control unit 31, a call control unit 32, an input control unit 33, a six-axis sensor control unit 34, a step marker extraction unit 35, a step marker right-and-left determination unit 36, a walking posture locus amount calculation unit 37, a walking posture diagnostic unit 38, and a display control unit 39. Further, the walking posture locus amount calculation unit 37 includes a front-back turning amount calculation unit 37A, a trunk turning amount calculation unit 37B, a right-and-left turning amount calculation unit 37C, a right-and-left moving distance calculation unit 37D, an up-and-down moving distance calculation unit 37E, and a landing impact calculation unit 37F. Note that the control unit 25 is, for example, an integrated circuit such as an application specific integrated circuit (ASIC) and a field programmable gate array (FPGA), and an electronic circuit such as a central processing unit (CPU) and a micro processing unit (MPU).

The storage unit 26 includes a diagnostic threshold value table 41, a diagnostic advice table 42, a diagnostic history table 43, and a data storage unit 44. Note that the storage unit 26 is, for example, a semiconductor memory device such as a random access memory (RAM) and a flash memory, and a storage device such as hard disk and an optical disk.

The radio communication unit 21 performs radio communication with a mobile phone network. For example, the radio communication unit 21 is a base band processor and the like that performs a process in relation to communication or a telephone call in a wideband code division multiple access (W-CDNtA).

The input unit 22 is an input device for inputting various types of information and for operating instructions, and is, for examples, a numerical keypad for inputting a number, a character, and the like, and a cursor key used for menu selection, display scroll, and the like. The display unit 23 is an output device for outputting various types of information, and is, for examples, a crystal liquid display and a speaker.

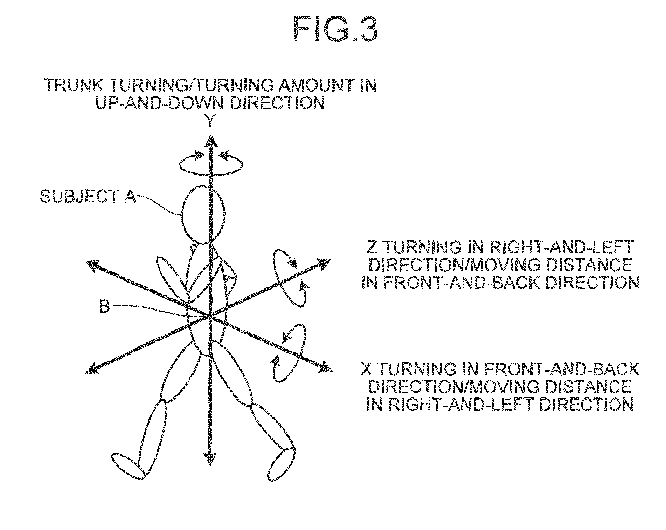

The six-axis sensor 24 includes a triaxial acceleration sensor that detects acceleration in the directions of mutually-perpendicular three axes and a triaxial angular velocity sensor (gyro sensor) that detects turning angular velocity around mutually-perpendicular three axes. Here, a measurement amount measured by the six-axis sensor 24 will be described with reference to FIG. 3. FIG. 3 is a diagram illustrating a measurement amount of the six-axis sensor. As illustrated in FIG. 3, an attached position of the six-axis sensor 24 is a waist portion B of a subject A. The six-axis sensor 24 is attached to the subject A in such a way that coordinate axes included in its own device coincide with reference coordinate axes where an x-axis is a right-and-left direction of the subject A, a y-axis is an up-and-down direction of the subject A, and a z-axis is a front-and-back direction of the subject A as the reference coordinate axes.

The triaxial acceleration sensor included in the six-axis sensor 24 detects acceleration in the directions of the mutually-perpendicular three axes. The acceleration in the x-axis direction serves as a displacement value associated with a movement in the right-and-left direction in walking of the subject A. That is, the acceleration serves as a moving distance in the right-and-left direction in reference to an attached posture of the six-axis sensor 24 at a predetermined timing, and a leftward moving distance indicates plus and a rightward moving distance indicates minus. The acceleration in the y-axis direction serves as a displacement value associated with a movement in the up-and-down direction in walking of the subject A. That is, the acceleration serves as a moving distance in the up-and-down direction in reference to the attached posture of the six-axis sensor 24 at a predetermined timing, and an upward moving distance indicates plus and a downward moving distance indicates minus. The acceleration in the z-axis direction serves as a displacement value associated with a movement in the front-and-back direction in walking of the subject A. That is, the acceleration serves as a moving distance in the front-and-back direction in reference to the attached posture of the six-axis sensor 24 at a predetermined timing, and a forward moving distance indicates plus and a backward moving distance indicates minus.

Also, the triaxial angular velocity sensor included in the six-axis sensor 24 detects angular velocity around the mutually-perpendicular three axes. The angular velocity around the x-axis serves as a displacement value associated with turning in the front-and-back direction in walking of the subject A. That is, the angular velocity serves as a turning amount in the front-and-back direction in reference to an attached posture of the six-axis sensor 24 at a predetermined timing, and a backward turning amount (in a rising direction) indicates plus and a forward turning amount (in a bending-forward direction) indicates minus. The angular velocity around the y-axis serves as a displacement value associated with turning in a trunk direction in walking of the subject A. That is, the angular velocity serves as a turning amount in the right-and-left direction in reference to the attached posture of the six-axis sensor 24 at a predetermined timing, and a rightward turning amount indicates plus and a leftward turning amount indicates minus. The angular velocity around the z-axis serves as a displacement value associated with turning in the right-and-left direction in walking of the subject A. That is, the angular velocity serves as a turning amount in the right-and-left direction in reference to the attached posture of the six-axis sensor 24 at a predetermined timing, and a rightward turning amount indicates plus and a leftward turning amount indicates minus.

Referring back to FIG. 2, the radio control unit 31 controls radio communication by the radio communication unit 21. The call control unit 32 controls a call in relation to the radio communication controlled by the radio control unit 31.

The input control unit 33 performs various types of input control. To be specific, when obtaining a start instruction of a walking posture diagnosis from the input unit 22, the input control unit 33 outputs the start instruction of a walking posture diagnosis to the six-axis sensor control unit 34 and the step marker extraction unit 35.

The six-axis sensor control unit 34 obtains various types of data output from the six-axis sensor 24 and stores the obtained various types of data in the data storage unit 44 as raw data. To be specific, when obtaining the start instruction of a walking posture diagnosis from the input control unit 33, the six-axis sensor control unit 34 obtains the various types of data of a specified period by predetermined samples in one second from the six-axis sensor 24 based on the obtained instruction, and stores it in the data storage unit 44 as the raw data. For example, the six-axis sensor control unit 34 obtains the various types of data of one hour by 60 samples in one second where the specified period is one hour. Note that the various types of data include a moving distance in the right-and-left direction, a moving distance in the up-and-down direction, a moving distance in the front-and-back direction, a turning amount in the front-and-back direction, a turning amount of a trunk, and a turning amount in the right-and-left direction. Then, the various types of data of each timing are stored as the raw data.

The step marker extraction unit 35 extracts a predetermined characteristic timing that appears at every step as a walking timing (hereinafter, referred to as "step marker") based on the acceleration in the y-axis direction, that is, the moving distance in the up-and-down direction. Here, the step marker is described as a marker at a timing of landing of a step (a timing when a heel of a foot touches on the ground). To be specific, the step marker extraction unit 35 refers to the upward and downward moving distances in the raw data stored in the data storage unit 44 and selects a downward (minus) turning-back timing from among the upward and downward moving distances at a plurality of timings.

Also, the step marker extraction unit 35 determines whether the moving distance at the selected turning-back timing is smaller than the moving distance at the timing of five samples earlier than the selected turning-back timing, and the moving distance at the selected turning-back timing is smaller than the moving distance at the timing of five samples later than the selected turning-back timing. Then, the step marker extraction unit 35 extracts the selected turning-back timing as a step marker when the moving distance at the selected turning-back timing is smaller than the moving distance at the five-sample-earlier timing, and is smaller than the moving distance at the five-sample-later timing.

Further, the step marker extraction unit 35 determines whether the extracted step marker is less than 250 milliseconds from an immediately preceding step marker. Then, when determining that the extracted step marker is less than 250 milliseconds from the immediately preceding step marker, the step marker extraction unit 35 determines that the extracted step marker is not a step of a walking posture and is therefore not a step marker. Then, the step marker extraction unit 35 outputs raw data, to which a distinction that indicates whether a step marker is added, to the step marker right-and-left determination unit 36.

Note that the step marker extraction unit 35 performs determination of a step of a walking posture using, but not limited to, a lower limit, and may perform the determination further using an upper limit. When performing the determination using the upper limit, for example, the step marker extraction unit 35 determines whether the extracted step marker exceeds 1000 milliseconds from the immediately preceding step marker. Then, when determining that the extracted step marker exceeds 1000 milliseconds from the immediately preceding step marker, the step marker extraction unit 35 determines that the extracted step marker is not a step of the walking posture and is therefore not a step marker.

The step marker right-and-left determination unit 36 determines a right/left foot that constitutes a step of a step marker based on the angular velocity around the z-axis around the step marker, that is, the right/left direction (plus/minus sign) of a maximum turning amount in the right-and-left direction. To be specific, the step marker right-and-left determination unit 36 selects a step marker that the right/left foot is to be determined, and immediately preceding and immediately subsequent step markers from the raw data output from the step marker extraction unit 35. Note that, here, the step marker that the right or left foot is to be determined is referred to as a "right-and-left determination step marker". Then, the step marker right-and-left determination unit 36 calculates a preceding period between the right-and-left determination step marker and the immediately preceding step marker and a subsequent period between the right-and-left determination step marker and the immediately subsequent step marker. Then, the step marker right-and-left determination unit 36 calculates a range from 30% of the preceding period to 30% of the subsequent period around the right-and-left determination step marker as a preceding-and-subsequent search range.

Further, the step marker right-and-left determination unit 36 reads out a maximum turning amount in the right-and-left direction in the calculated preceding-and-subsequent search range from the raw data. Also, the step marker right-and-left determination unit 36 determines whether the read out maximum turning amount indicates the plus direction. Then, when determining that the read out maximum turning amount indicates the plus direction, the step marker right-and-left determination unit 36 determines that it is a rightward turning and is a left foot landing. Meanwhile, when determining that the read out maximum turning amount indicates the minus direction, the step marker right-and-left determination unit 36 determines that it is a leftward turning and is a right foot landing. Then, the step marker right-and-left determination unit 36 adds a distinction that indicates the right/left foot that constitutes a step to data of the step marker in the raw data, and outputs the added data to the walking posture locus amount calculation unit 37.

The front-back turning amount calculation unit 37A selects successive step markers and calculates a moving locus caused by the walking of the selected step markers based on the angular velocity around the x-axis, that is, turning amounts in the front-and-back direction. To be specific, the front-back turning amount calculation unit 37A selects three successive step markers from the output data output from the step marker right-and-left determination unit 36. Then, the front-back turning amount calculation unit 37A calculates a preceding period between a central step marker and an immediately preceding step marker and a subsequent period between the central step marker and an immediately subsequent step marker. Then, the front-back turning amount calculation unit 37A calculates a range from 50% of the preceding period to 20% of the subsequent period around the central step marker as the preceding-and-subsequent search range. Also, the front-back turning amount calculation unit 37A shifts the center of step marker to the immediately subsequent step marker, and calculates the preceding-and-subsequent search range around the new central step marker.

Further, the front-back turning amount calculation unit 37A specifies each backward maximum turning amount that indicates plus (in the rising direction) from each preceding-and-subsequent search range based on the turning amounts in the front-and-back direction of the output data. Further, the front-back turning amount calculation unit 37A specifies a forward maximum turning amount (in the bending-forward direction) in a period having successive maximum turning amounts among the specified backward maximum turning amounts. Then, regarding the successive backward maximum turning amounts (in the rising direction) and the forward maximum turning amount (in the bending-forward direction), the front-back turning amount calculation unit 37A calculates an absolute value of a difference between the backward maximum turning amount and the forward maximum turning amount as a "rising turning amount" when the timing of becoming the backward direction comes later than the timing of becoming the forward direction. Meanwhile, regarding the successive backward maximum turning amounts (in the rising direction) and the forward maximum turning amount (in the bending-forward direction), the front-back turning amount calculation unit 37A calculates an absolute value of a difference between the backward maximum turning amount and the forward maximum turning amount as a "bending-forward turning amount" when the timing of becoming the backward direction comes earlier than the timing of becoming the forward direction.

The trunk turning amount calculation unit 37B selects successive step markers and calculates a moving locus caused by the walking of the selected step markers based on the angular velocity around the y-axis, that is, turning amounts in a trunk direction. To be specific, the trunk turning amount calculation unit 37B selects three successive step markers from the output data output from the step marker right-and-left determination unit 36. Then, the trunk turning amount calculation unit 37B calculates a preceding period between a central step marker and an immediately preceding step marker and a subsequent period between the central step marker and an immediately subsequent step marker. Then, the trunk turning amount calculation unit 37B calculates a range from 30% of the preceding period to 30% of the subsequent period around the central step marker as the preceding-and-subsequent search range.

Also, the trunk turning amount calculation unit 37B reads out a maximum turning amount in the right-and-left direction in the calculated preceding-and-subsequent search range from the output data. Further, the trunk turning amount calculation unit 37B determines whether the read out maximum turning amount indicates the plus direction. Then, when determining that the read out maximum turning amount indicates the plus direction, the trunk turning amount calculation unit 37B determines that it is a rightward maximum turning amount. Meanwhile, when determining that the read out maximum turning amount indicates the minus direction, the trunk turning amount calculation unit 37B determines that it is a leftward maximum turning amount. Further, regarding successive rightward and leftward maximum turning amounts, the trunk turning amount calculation unit 37B calculates an absolute value of a difference between the rightward and leftward maximum turning amounts as a ""rightward trunk turning amount" when the timing of the rightward maximum turning amount comes earlier than the timing of the leftward maximum turning amount. Also, regarding the successive rightward and leftward maximum turning amounts, the trunk turning amount calculation unit 37B calculates an absolute value of a difference between the rightward and leftward maximum turning amounts as a "leftward trunk turning amount" when the timing of the rightward maximum turning amount comes later than the timing of the leftward maximum turning amount.

The right-and-left turning amount calculation unit 37C selects successive step markers and calculates a moving locus caused by the walking of the selected step markers based on the angular velocity around the z-axis, that is, turning amounts in the right-and-left direction. To be specific, the right-and-left turning amount calculation unit 37C selects two successive step markers from the output data output from the step marker right-and-left determination unit 36. Then, the right-and-left turning amount calculation unit 37C calculates a period between the selected two step markers as a step marker range.

Further, the right-and-left turning amount calculation unit 37C reads out a maximum turning amount in the right-and-left direction in the calculated step marker range from the output data. Also, the right-and-left turning amount calculation unit 37C determines whether the read out maximum turning amount indicates the plus direction. Then, when determining that the read out maximum turning amount indicates the plus direction, the right-and-left turning amount calculation unit 37C determines that it is a leftward maximum turning amount. Meanwhile, when determining that the read out maximum turning amount indicates the minus direction, the right-and-left turning amount calculation unit 37C determines that it is a leftward maximum turning amount. Also, regarding successive rightward and leftward maximum turning amounts, the right-and-left turning amount calculation unit 37C calculates an absolute value of a difference between the rightward and leftward maximum turning amounts as a "leftward turning amount" when the timing of the leftward maximum turning amount comes earlier than the timing of the rightward maximum turning amount. Also, regarding the successive rightward and leftward maximum turning amounts, the right-and-left turning amount calculation unit 37C calculates an absolute value of a difference between the rightward and leftward maximum turning amounts as a "rightward turning amount" when the timing of the leftward maximum turning amount comes later than the timing of the rightward maximum turning amount.

The right-and-left moving distance calculation unit 37D selects successive step markers and calculates a moving locus caused by the walking of the selected step markers based on the acceleration in the x-axis direction, that is, moving distances in the right-and-left direction. To be specific, the right-and-left moving distance calculation unit 37D selects three successive step markers from the output data output from the step marker right-and-left determination unit 36. Then, the right-and-left moving distance calculation unit 37D calculates a preceding period between a central step marker and an immediately preceding step marker and a subsequent period between the central step marker and an immediately subsequent step marker. Then, the right-and-left moving distance calculation unit 37D calculates a range from 30% of the preceding period to 30% of the subsequent period around the central step marker as the preceding-and-subsequent search range. Also, the right-and-left moving distance calculation unit 37D shifts the center of step marker to the immediately subsequent step marker and calculates the preceding-and-subsequent search range around the new central step marker.

Also, the right-and-left moving distance calculation unit 37D reads out a maximum moving distance in the right-and-left direction in each preceding-and-subsequent search range from the raw data. Also, the right-and-left moving distance calculation unit 37D determines whether the read out maximum moving distance indicates the plus direction. Then, when determining that the read out maximum moving distance indicates the plus direction, the right-and-left moving distance calculation unit 37D determines that it is a leftward maximum moving distance. Meanwhile, when determining that the read out maximum moving distance indicates the minus direction, the right-and-left moving distance calculation unit 37D determines that it is a rightward maximum moving distance. Also, regarding successive rightward and leftward maximum moving distances, the right-and-left moving distance calculation unit 37D calculates an absolute value of a difference between the rightward and leftward maximum moving distances as a "rightward moving distance" when the timing of the leftward maximum moving distance comes earlier than the timing of the rightward maximum moving distance. Also, regarding the successive rightward and leftward maximum moving distances, the right-and-left moving distance calculation unit 37D calculates an absolute value of a difference between the rightward and leftward maximum moving distances as a "leftward moving distance" when the timing of the leftward maximum moving distance comes later than the timing of the rightward maximum moving distance.

The up-and-down moving distance calculation unit 37E selects successive step markers and calculates a moving locus caused by the walking of the selected step markers based on the acceleration around the y-axis direction, that is, moving distances in the up-and-down direction. To be specific, the up-and-down moving distance calculation unit 37E selects two successive step markers from the output data output from the step marker right-and-left determination unit 36. Then, the up-and-down moving distance calculation unit 37E calculates a period between the selected two step markers as the step marker range.

Also, the up-and-down moving distance calculation unit 37E reads out an upward maximum moving distance in the calculated step marker range from the output data. Also, regarding successive upward and downward maximum moving distances, the up-and-down moving distance calculation unit 37E calculates an absolute value of a difference between the upward and downward maximum moving distances as an "upward moving distances" when the timing of the downward maximum moving distance comes earlier than the timing of the upward maximum moving distance. Also, regarding the successive upward and downward maximum moving distances, the up-and-down moving distance calculation unit 37E calculates an absolute value of a difference between the upward and downward maximum moving distances as a "downward moving distance" when the timing of the downward maximum moving distance comes later than the timing of the upward maximum moving distance.

The landing impact calculation unit 37F selects successive step markers and calculates impact of landing caused by the walking of the selected step markers based on the acceleration in the y-axis direction, that is, moving distances in the up-and-down direction. To be specific, the landing impact calculation unit 37F selects two successive step markers from the output data output from the step marker right-and-left determination unit 36. Then, the landing impact calculation unit 37F calculates a period between the selected two step markers as the step marker period.

Further, the landing impact calculation unit 37F reads out upward and downward maximum moving distances in the calculated step marker range from the output data. Also, the landing impact calculation unit 37F calculates an absolute value of a difference between the read out upward and downward maximum moving distances as an "impact at landing".

The walking posture diagnostic unit 38 diagnoses a walking posture based on various moving loci calculated by the walking posture locus amount calculation unit 37. To be specific, the walking posture diagnostic unit 38 obtains the various moving loci calculated by the walking posture locus amount calculation unit 37. Also, the walking posture diagnostic unit 38 calculates an average value for each moving locus using the obtained various moving loci. For example, the walking posture diagnostic unit 38 obtains a plurality of rightward turning amounts of a specified period calculated by the right-and-left turning amount calculation unit 37C and calculates the average value of the rightward turning amounts. Note that a calculation result of these various moving loci is referred to as "calculation data of various moving loci".

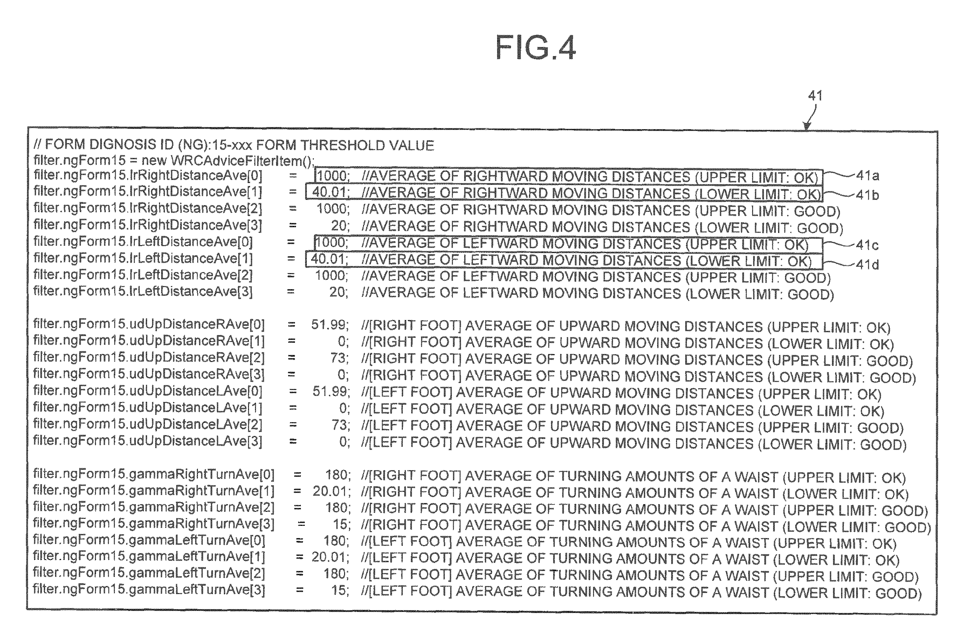

Further, the walking posture diagnostic unit 38 determines whether the various moving loci satisfy a reference in relation to a walking posture stored in the diagnostic threshold value table 41. Here, the diagnostic threshold value table 41 will be described with reference to FIG. 4. FIG. 4 is a diagram illustrating an example of a data structure of the diagnostic threshold value table. As illustrated in FIG. 4, the diagnostic threshold value table 41 stores a threshold value of a moving locus that serves as a reference in relation to a walking posture.
For example, the diagnostic threshold value table 41 stores, regarding the average value of the rightward moving distances, an upper limit 41a and a lower limit 41b that serve as the references. Also, the diagnostic threshold value table 41 stores, regarding an average value of leftward moving distances, an upper limit 41c and a lower limit 41d that serve as the references. Note that, in the example of FIG. 4, an average of rightward and leftward moving distances, an average of upward moving distances of the right and left feet, and an average of turning amounts of a waist of the right and left feet are stored. However, it is not limited to the above values, and a threshold value of a moving locus other than the above may be stored, so that the diagnosis of the walking posture can be further enhanced.

Referring back to FIG. 2, to be more specific, the walking posture diagnostic unit 38 calculates, when the moving locus is, for example, the rightward moving distances calculated by the right-and-left moving distance calculation unit 37D, an average value of the rightward moving distances. Then, the walking posture diagnostic unit 38 determines that the reference in relation to the walking posture is satisfied when the average value of the rightward moving distances is the lower limit 41b or more, which is a lower limit of the average of the rightward moving distances, and is the upper limit 41a or less, which is an upper limit of the average of the rightward moving distances, and which are stored in the diagnostic threshold value table 41. Meanwhile, the walking posture diagnostic unit 38 determines that the reference in relation to the walking posture is not satisfied when the average value of the rightward moving distances is less than the lower limit 41b serving as a lower limit of the average of the rightward moving distances, or exceeds the upper limit 41a, which are stored in the diagnostic threshold value table 41. Then, the walking posture diagnostic unit 38 calculates a score obtained from the average value of the rightward moving distances based on a determination result. For example, when the reference in relation to the walking posture is satisfied, the walking posture diagnostic unit 38 gives a perfect score to the score allocated to the rightward moving distance among the scores allocated to the various moving loci. Meanwhile, the walking posture diagnostic unit 38 deducts a value in accordance with a difference value between the average value of the rightward moving distances and the upper limit 41a or the lower limit 41b from the score allocated to the rightward moving distance among the scores allocated to the various moving loci when the reference in relation to the walking posture is not satisfied. Then, the walking posture diagnostic unit 38 calculates a score of each of the various moving loci and sums up the calculated scores. Note that up to 100 points may be given when all of the scores allocated to the various moving loci are summed up. Also, each score allocated to the various moving loci may be weighted according to the degree of importance of the various moving loci, so that the diagnosis of the walking posture can be more accurate.

Also, the walking posture diagnostic unit 38 creates diagnostic form (walking posture) information based on a comparison result between the data stored in the diagnostic threshold value table 41 and the various moving loci. The diagnostic form (walking posture) information includes, for example, a diagnostic point in relation to a form and an identification number in the diagnostic advice table 42 described below. Also, the walking posture diagnostic unit 38 combines the diagnostic form (walking posture) information and the score with the "calculation data of various moving loci" and outputs it to the display control unit 39.

The display control unit 39 displays a diagnosis result diagnosed by the walking posture diagnostic unit 38 on the display unit 23. To be specific, the display control unit 39 obtains the "calculation data of various moving loci" in which the diagnostic form (walking posture) information and the scores are combined from the walking posture diagnostic unit 38. Also, the display control unit 39 edits the "calculation data of various moving loci" based on the data stored in the diagnostic advice table 42 and displays it on the display unit 23. Also, the display control unit 39 stores the "calculation data of various moving loci" and an advice content in the diagnostic history table 43.

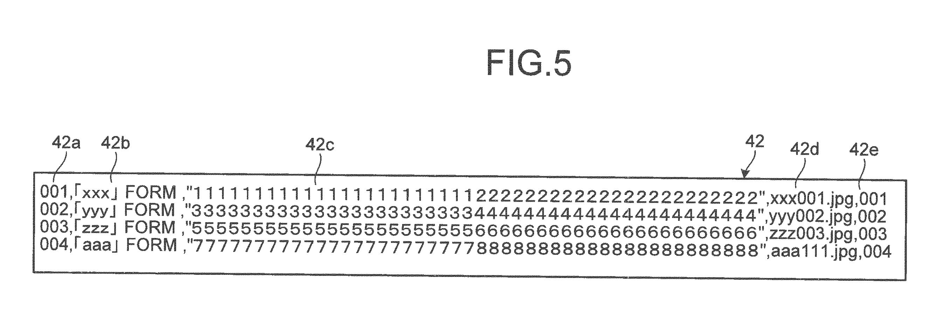

Here, the diagnostic advice table 42 will be described with reference to FIG. 5. FIG. 5 is a diagram illustrating an example of a data structure of the diagnostic advice table. As illustrated in FIG. 5, the diagnostic advice table 42 stores a measurement point 42b, a message content 42c, a photograph content 42d, and an image id 42e in association with each other for each identification number 42a. The identification number 42a is an identification number of diagnostic advice and corresponds to the identification number in the diagnostic form (walking posture) information. The measurement point 42b is an advice point, and a diagnostic point in relation to a form in the diagnostic form (walking posture) information is embedded therein. The message content 42c is a content about one point advice.

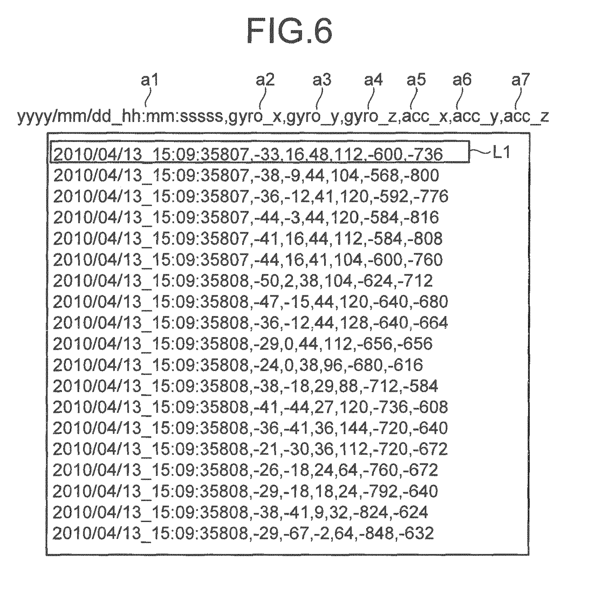

Next, a structure of the raw data stored in the data storage unit 44 by the six-axis sensor control unit 34 will be described with reference to FIG. 6. FIG. 6 is a diagram illustrating an example of data (raw data) output from the six-axis sensor 24. As illustrated in FIG. 6, the raw data including a turning amount a2 in the front-and-back direction, a turning amount a3 in the trunk direction, a turning amount a4 in the right-and-left direction, a moving distance a5 in the right-and-left direction, a moving distance a6 in the up-and-down direction, and a moving distance a7 in the front-and-back direction are stored in association with each other in a line for each year-month-day-time a1 detected by the six-axis sensor 24.

In the example of FIG. 6, as illustrated in line L1, the turning amount a2 in the front-and-back direction is "-33", the turning amount a3 in the trunk direction is "16", the turning amount a4 in the right-and-left direction is "48", and the moving distance a5 in the right-and-left direction is "112" at 15:09 in 2010/4/13. Further, the moving distance a6 in the up-and-down direction is "-600" and the moving distance a7 in the front-and-back direction is "-736". Note that a start of the year-month-day-time a1 is when an execution instruction of a walking posture is given. Also, the unit of the turning amount is expressed in degree/second and the unit of the moving distance is expressed in millimeter (mm).

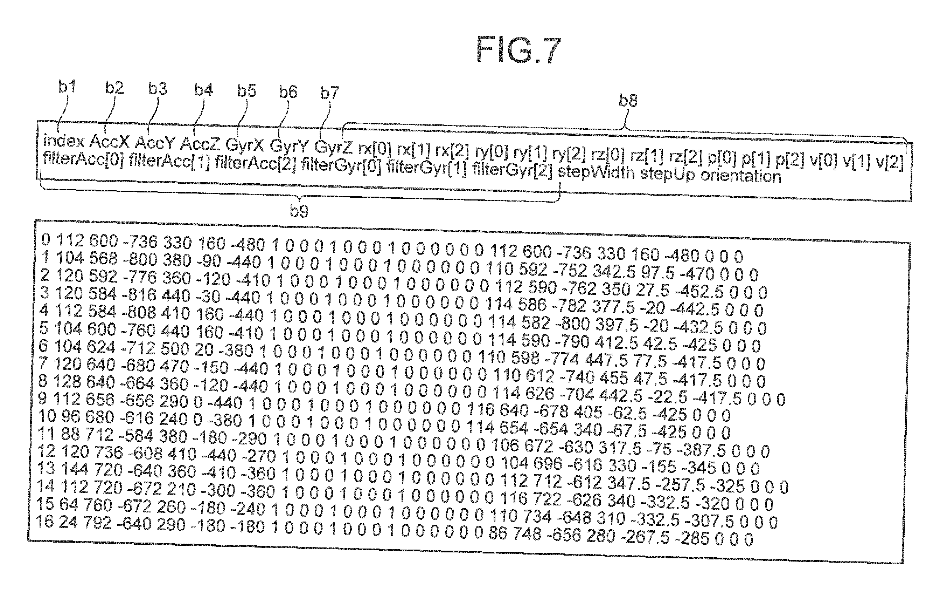

Next, a data structure temporarily used in a step marker extraction process of the step marker extraction unit 35 will be described with reference to FIG. 7. FIG. 7 is a diagram illustrating an example of data temporarily used in the step marker extraction process. As illustrated in FIG. 7, the data including a moving distance b2 in the right-and-left direction, a moving distance b3 in the up-and-down direction, a moving distance b4 in the front-and-back direction, a turning amount b5 in the front-and-back direction, a turning amount b6 in the trunk direction, a turning amount b7 in the right-and-left direction, each element value b8 in a turning matrix that expresses a posture of a mobile phone, a filtering b9 of each moving distance and turning amount are stored in a line for each index b1 in association with each other. Note that the index b1 corresponds to each year-month-day-time a1 included in the raw data, and is "0" at the start of a diagnosis, for example.

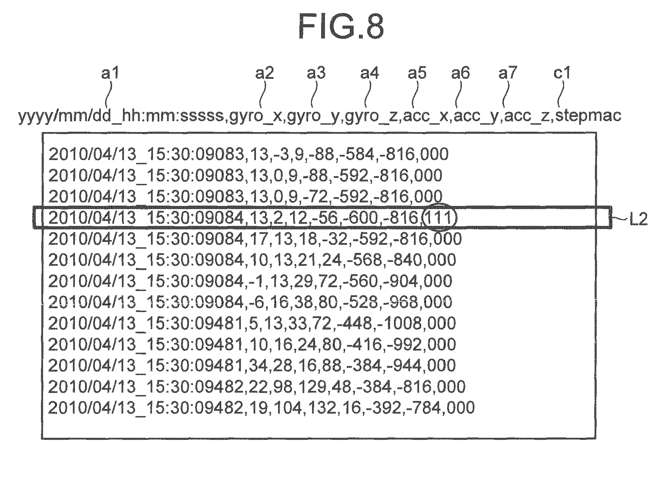

Next, a structure of output data output to the step marker right-and-left determination unit 36 by the step marker extraction process of the step marker extraction unit 35 will be described with reference to FIG. 8. FIG. 8 is a diagram illustrating an example of data output by the step marker extraction process. As illustrated in FIG. 8, the output data including the turning amount a2 in the front-back direction, the turning amount a3 in the trunk direction, the turning amount a4 in the right-and-left direction, the moving distance a5 in the right-and-left direction, the moving distance a6 in the up-and-down direction, the moving distance a7 in the front-back direction, and a distinction c1 of a step marker are stored in a line for each year-month-day-time a1 in association with each other That is, the output data is data to which the distinction c1 that indicates whether it is a step marker is added in each line in the raw data. In the example of FIG. 8, as illustrated in line L2, "111" that indicates it is a step marker is added. Also, "000" that indicates it is not a step marker is added to lines other than line L2.

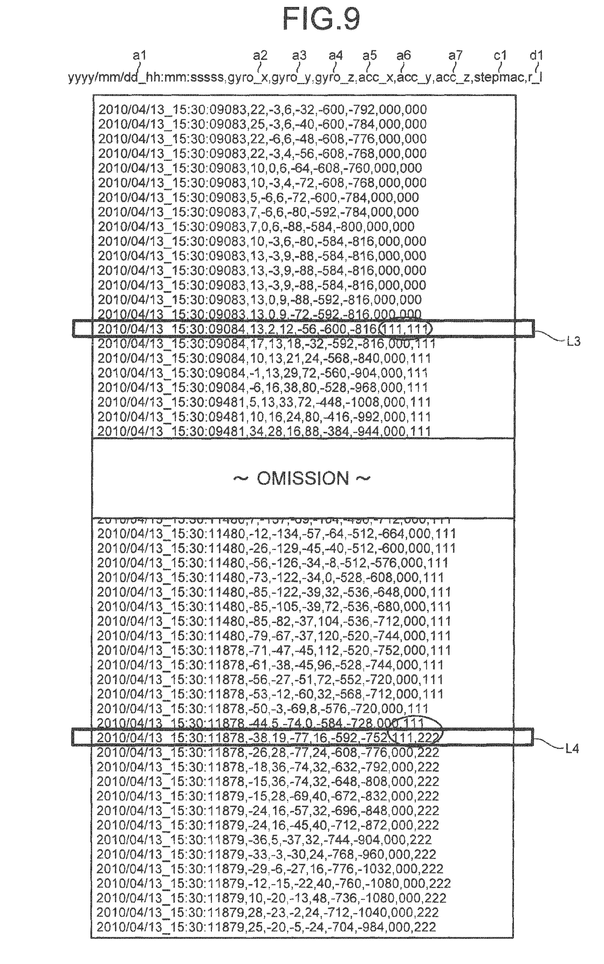

Next, a structure of output data output to the walking posture locus amount calculation unit 37 by a step marker right-and-left determination process of the step marker right-and-left determination unit 36 will be described with reference to FIG. 9. FIG. 9 is a diagram illustrating an example of data output by the step marker right-and-left determination process. As illustrated in FIG. 9, the output data including the turning amount a2 in the front-back direction, the turning amount a3 in the trunk direction, the turning amount a4 in the right-and-left direction, the moving distance a5 in the right-and-left direction, the moving distance a6 in the up-and-down direction, and the moving distance a7 in the front-back direction are stored in association with each other for each year-month-day-time a1 Also, the output data including the distinction c1 of a step marker and a distinction d1 indicating whether a right foot landing or a left foot landing are stored in association with each other in each line. That is, the output data is data into which the distinction d1 of a right/left foot that constitute a step is added in each line in the raw data.

In the example of FIG. 9, as illustrated in line L3, "111" that indicates it is the left foot landing is added to the distinction d1 of a right/left foot. Also, as illustrated in line L4, "222" that indicates the right foot landing is added to the distinction d1 of a right/left foot. Also, in lines L3 to L4, "111" that indicates the left foot landing is added to the distinction d1 of a right/left foot. That is, this period expresses a moving locus caused by a step of a left foot. Also, after line L4, "222" that indicates the right foot landing is added to the distinction d1 of a right/left foot. That is, this period expresses a moving locus caused by a step of a right foot.

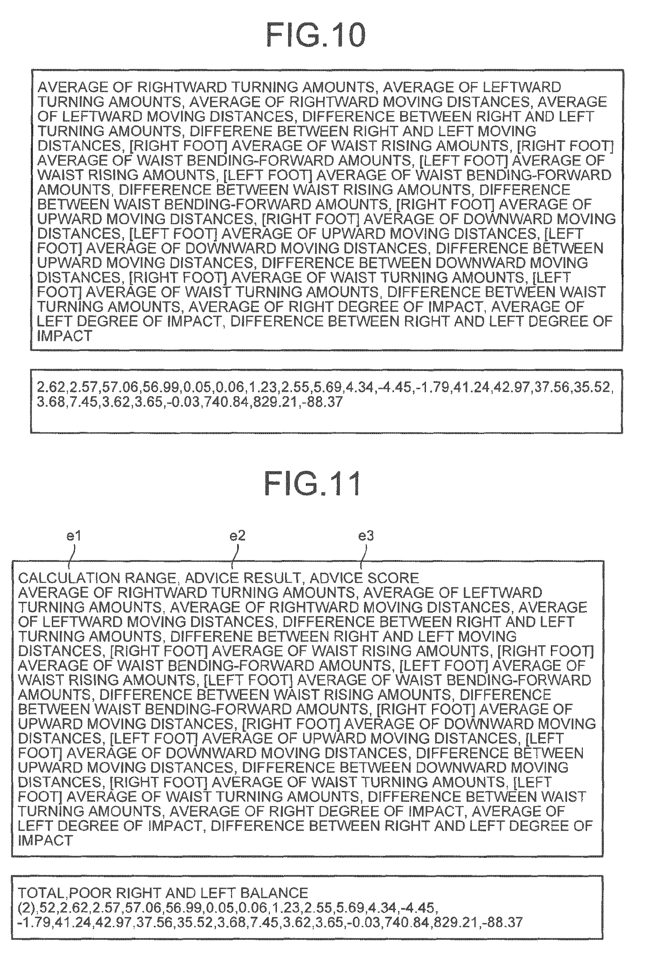

Next, a structure of calculation data of the various moving loci using a walking posture locus amount calculation result calculated by the walking posture diagnostic unit 38 will be described with reference to FIGS. 10 and 11. FIG. 10 is an example of a case with no score and diagnostic form, and FIG. 11 is an example of a case with a score and diagnostic form in the calculation data of the various moving loci using the walking posture locus amount calculation process result.

As illustrated in FIG. 10, the calculation data includes averages of rightward turning amounts, leftward turning amounts, rightward moving distances, and leftward moving distances, a difference between the rightward and leftward turning amounts, and a difference between the rightward and leftward moving distances. Also, the calculation data includes an average of rising amounts and an average of bending-forward amounts of a waist in the right foot landing, an average of rising amounts and an average of bending-forward amounts of a waits in the left foot landing, a difference between the rising amounts of a waist, and a difference between the bending-forward amounts of a waist. Also, the calculation data includes an average of upward moving distances in the right foot landing, an average of downward moving distances in the right foot landing, an average of upward moving distances in the left foot landing, an average of downward moving distances in the left foot landing, a difference between the upward moving distances, and a difference between the downward moving distances. Also, the calculation data includes an average of trunk turning amounts of a waist in the right foot landing, an average of trunk turning amounts of a waist in the left foot landing, and a difference between the trunk turning amounts of a waist. Further, the calculation data includes an average of the degree of impact of a right foot, an average of the degree of impact of a left foot, and a difference between the degree of impact of the right and left feet.

Also, as illustrated in FIG. 11, the calculation data is data in which a calculation range e1, an advice result e2, and an advice score e3 are added to the calculation data of the various moving loci illustrated in FIG. 10. The calculation range e1 represents attribute that indicates which range of data in the raw data is used for calculation, and for example, includes "total" indicating the entire period range, "Start" indicating a predetermined period range from a start of a diagnosis among from the entire period range, and "Middle" indicating a middle range. The advice result e2 represents the diagnostic form (walking posture) information. The advice score e3 represents a score calculated based on the calculation data of the various moving loci. Note that the calculation range e1 may represent a parameter of a start instruction of a diagnosis.

In the example of FIG. 11, "Total" indicating that data in the entire period range of the raw data is used is presented in the calculation range e1. In the advice result e2, "Poor right and left balance (2)" is presented. In the advice score e3, "52.2" points is presented. Note that the numerical value in the bracket of the advice result e2 serves as the identification number in the diagnostic advice table 42.

### [A specific example of step marker extraction]

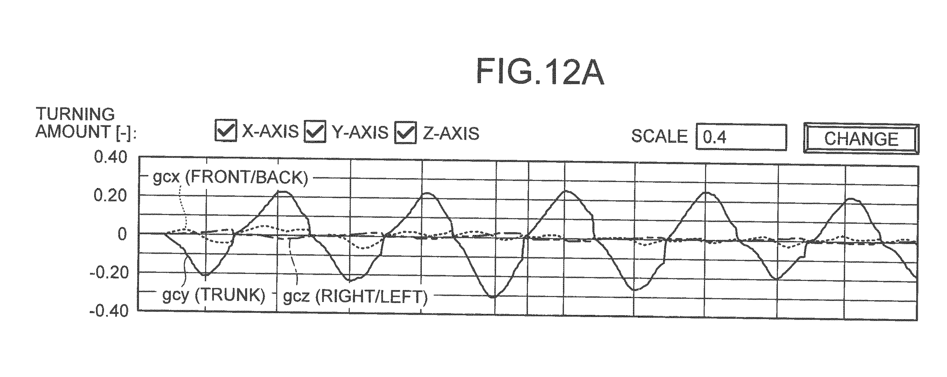

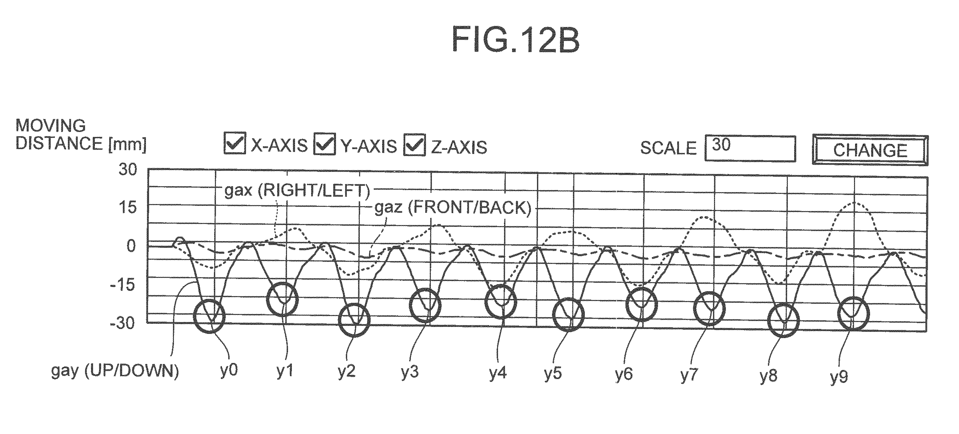

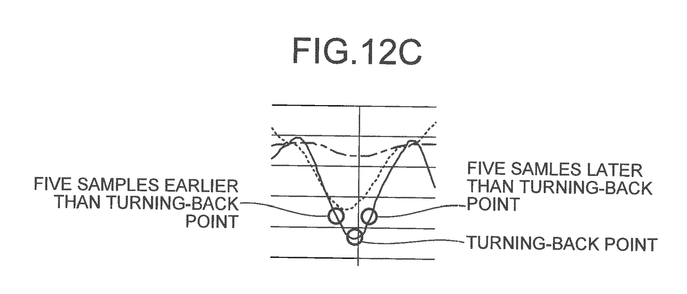

Next, a specific example of step marker extraction by the step marker extraction unit 35 will be described with reference to FIGS. 12A to 12C. FIGS. 12A to FIG. 12C are explanation diagrams describing a specific example of the step marker extraction. FIG. 12A is a graph illustrating the various turning amounts of each time, FIG. 12B is a graph illustrating the various moving distances of each time, and FIG. 12C is a part of a graph illustrating a step marker extraction method.

The horizontal axis of FIG. 12A represents a time axis and the vertical axis represents various turning amounts (unit: degree) illustrated based on the raw data. Forward and backward turning amounts gcx expressed in the x-axis of the reference coordinate axis among the various turning amounts are illustrated with a broken line, trunk turning amounts gcy expressed in the y-axis are illustrated with a solid line, and rightward and leftward turning amounts gcz expressed in the z-axis are illustrated with a dashed line.

The horizontal axis of FIG. 12B represents a time axis and the vertical axis represents the various moving distances (unit: mm) illustrated based on the raw data. Rightward and leftward moving distances gax expressed in the x-axis of the reference coordinate axis among the various moving distances are illustrated in a broken line, upward and downward moving distances gay expressed in the y-axis are illustrated in a solid line, and forward and backward moving distances gaz expressed in the z-axis are illustrated in a dashed line. The step marker extraction by the step marker extraction unit 35 uses the upward and downward moving distances gay from among the various turning amounts and moving distances. Among the upward and downward moving distances gay, an upward moving distance indicates plus and a downward moving distance indicates minus. Note that a reference of the upward and downward moving distances is, for example, an attached posture at a start timing of a walking posture diagnosis of a mobile phone to which a six-axis sensor 24 is equipped.

The step marker extraction unit 35 refers to the upward and downward moving distances gay and selects turning-back timings in the minus direction. Here, the step marker extraction unit 35 selects timings illustrated by y0 to y9 as the turning-back timings.

As illustrated in FIG. 12C, it is determined whether the turning-back point is a step marker. That is, the step marker extraction unit 35 refers to a moving distance five samples earlier than the moving distance at the selected turning-back timing and a moving distance five samples later than the moving distance at the selected turning-back timing. Then, the step marker extraction unit 35 determines whether the moving distance at the turning-back timing is smaller than the moving distance at the five-sample-earlier timing and is smaller than the moving distance at the five-sample-later timing. Then, when the moving distance at the turning-back timing is smaller than the moving distance at the five-sample-earlier timing and is smaller than the moving distance at the five-sample-later timing, the step marker extraction unit 35 extracts this turning-back timing as a step marker.

### [A specific example of step marker right-and-left determination]

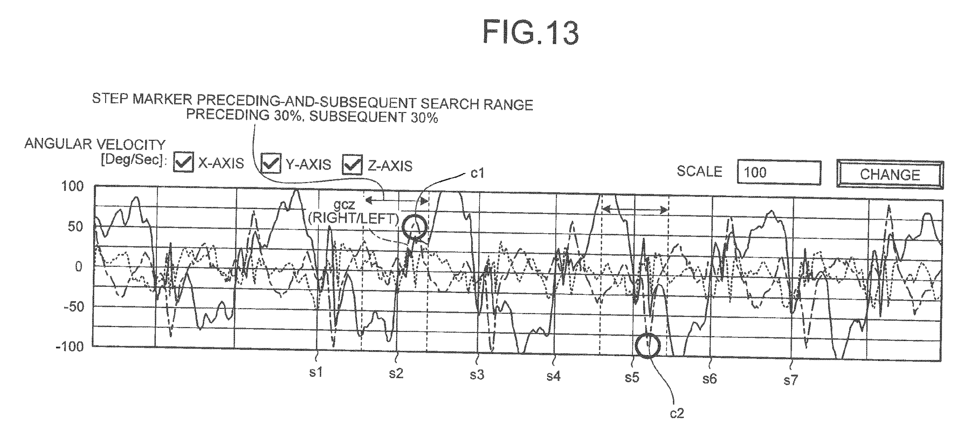

Next, a specific example of step marker right-and-left determination by the step marker right-and-left determination unit 36 will be described with reference to FIG. 13. FIG. 13 is an explanation diagram describing a specific example of the step marker right-and-left determination. The step marker right-and-left determination by the step marker right-and-left determination unit 36 uses the rightward and leftward turning amounts gcz from among various turning amounts and moving distances. Among the rightward and leftward turning amounts gcz, a rightward turning amount indicates plus and a leftward turning amount indicates minus. Note that a reference of the rightward and leftward turning amounts is, for example, an attached posture at a start timing of a walking posture diagnosis of a mobile phone to which a six-axis sensor 24 is equipped.

The step marker right-and-left determination unit 36 selects a step marker (right-and-left determination step marker) that determines the right/left foot, and immediately preceding and immediately subsequent step markers of the step marker. Here, the right-and-left determination step marker is s2, and the immediately preceding and immediately subsequent step markers of this step marker are respectively s1 and s3. Then, the step marker right-and-left determination unit 36 calculate a preceding period between s1 and s2 and a subsequent period between s2 and s3, and calculates a range from 30% of the preceding period to 30% of the subsequent period around s2 as the preceding-and-subsequent search range. The right/left foot that constitutes a step in the right-and-left determination step marker s2 is determined within this preceding-and-subsequent search range around the right-and-left determination step marker s2.

Then, the step marker right-and-left determination unit 36 selects a maximum turning amount in the right-and-left direction from the preceding-and-subsequent search range and determines whether the selected maximum turning amount indicates the plus direction. Then, when determining the maximum turning amount indicates the plus direction, the step marker right-and-left determination unit 36 determines that it is a rightward turning and is a left foot landing. Here, a maximum turning amount c1 in the right-and-left direction from the preceding-and-subsequent search range around the right-and-left determination step markers s2 is selected and it is determined that it is the left foot landing because c1 indicates the plus direction.

Meanwhile, when determining that the maximum turning amount indicates the minus direction, the step marker right-and-left determination unit 36 determines that it is a right foot landing. For example, when the right-and-left determination step marker is s5, a maximum turning amount c2 in the right-and-left direction is selected from the preceding-and-subsequent search range, which is calculated in a similar manner, and it is determined that it is the right foot landing because c2 indicates the minus direction.

### [A specific example of front-back turning amount calculation]

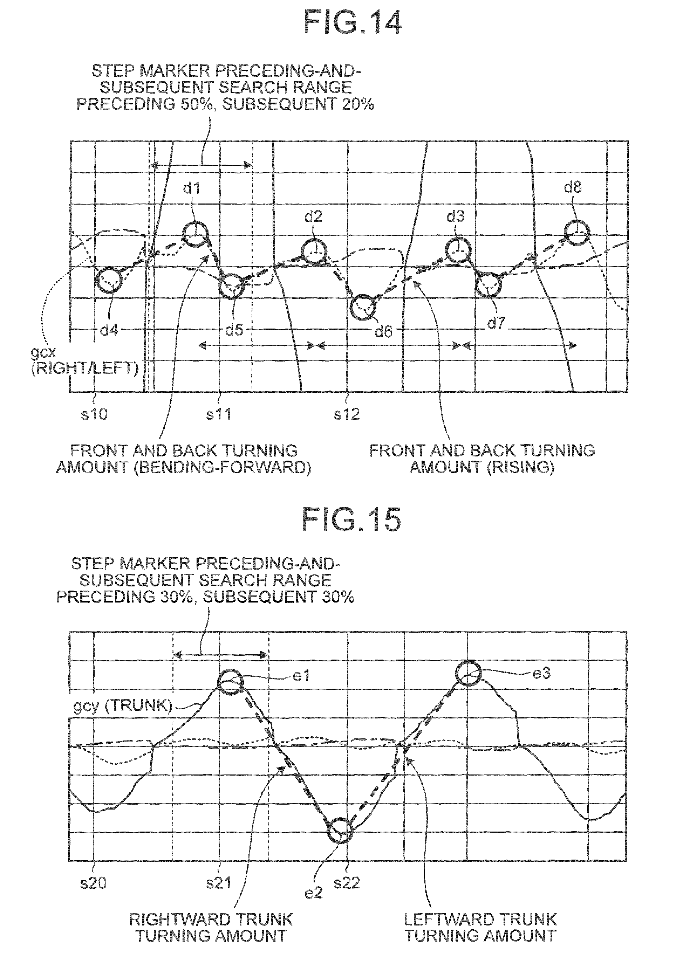

Next, a specific example of front-back turning amount calculation by the front-back turning amount calculation unit 37A will be described with reference to FIG. 14. FIG. 14 is an explanation diagram describing a specific example of the front-back turning amount calculation. The front-back turning amount calculation by the front-back turning amount calculation unit 37A uses the forward and backward turning amounts gcx from among the various turning amounts and moving distances. Among the forward and backward turning amounts gcx, a backward turning amount (in the rising direction) indicates plus and a forward turning amount (in the bending-forward direction) indicates minus. Note that a reference of the forward and backward turning amounts is, for example, an attached posture at a start timing of a walking posture diagnosis of a mobile phone to which a six-axis sensor 24 is equipped.

The front-back turning amount calculation unit 37A selects three successive step markers. Then, the front-back turning amount calculation unit 37A calculates a preceding period between a central step marker and an immediately preceding step marker and a subsequent period between the central step marker and an immediately subsequent step marker. Then, the front-back turning amount calculation unit 37A calculates a range from 50% of the preceding period to 20% of the subsequent period around the central step marker as the preceding-and-subsequent search range. For example, when the central step marker is s11, the preceding-and-subsequent search range is calculated using an immediately preceding step marker s10 and an immediately subsequent step marker s12.

Then, the front-back turning amount calculation unit 37A specifies a plus maximum turning amount in the preceding-and-subsequent search range. Here, the timings of d1, d2, and d3 are specified as the timings of the plus maximum turning amount. Then, the front-back turning amount calculation unit 37A specifies a minus maximum turning amount in a period having successive maximum turning amounts among the plus maximum turning amounts. Here, the timing of d5 in the period between d1 and d2 and the timing d6 in the period between d2 and d3 are specified as the timings of the minus maximum turning amount. Then, regarding successive plus and minus maximum turning amounts, the front-back turning amount calculation unit 37A calculates an absolute value of a difference between the plus and minus maximum turning amounts as a "rising turning amount" when the timing of the plus maximum turning amount comes later than the timing of the minus maximum turning amount. For example, the "rising turning amount" is the absolute value of the difference between the minus maximum turning amount at the timing of d6 and the plus maximum turning amount at the timing of d3.

Meanwhile, regarding the successive plus and minus maximum turning amounts, the front-back turning amount calculation unit 37A calculates an absolute value of a difference between the plus and minus maximum turning amounts as a "bending-forward turning amount" when the timing of the plus maximum turning amount comes earlier than the timing of the minus maximum turning amount. For example, the "bending-forward turning amount" is the absolute value of the difference between the maximum turning amount at the timing of d1 and the maximum turning amount at the timing of d5.

### [A specific example of trunk turning amount calculation]

Next, a specific example of trunk turning amount calculation by the trunk turning amount calculation unit 37B will be described with reference to FIG. 15. FIG. 15 is an explanation diagram describing a specific example of the trunk turning amount calculation. The trunk turning amount calculation by the trunk turning amount calculation unit 37B uses the trunk turning amounts gcy from among the various turning amounts and moving distances. Among the trunk turning amounts gcy, a rightward turning amount indicates plus and a leftward turning amount indicates minus. Note that a reference of the turning amount in the trunk direction is, for example, an attached posture at a start timing of a walking posture diagnosis of a mobile phone to which a six-axis sensor 24 is equipped. Also, description of a method of calculating the preceding-and-subsequent search range is omitted because it is similar to the step marker right-and-left determination unit 36. In the example of FIG. 15, when the central step marker is s21, the preceding-and-subsequent search range is calculated using an immediately preceding step markers s20 and an immediately subsequent step markers s22.

The trunk turning amount calculation unit 37B specifies a turning-back point of each trunk turning amount from the preceding-and-subsequent search range after the calculation of the preceding-and-subsequent search range. That is, the trunk turning amount calculation unit 37B specifies a plus maximum turning amount or a minus maximum turning amount from the preceding-and-subsequent search range. Here, the timings of e1 and e3 are specified as the timings of the plus maximum turning amounts and the timing of e2 is specified as the timing of the minus maximum turning amount. Then, regarding successive plus and minus maximum turning amounts, the trunk turning amount calculation unit 37B calculates an absolute value of a difference between the plus and minus maximum turning amounts as a "leftward trunk turning amount" when the timing of the plus maximum turning amount comes later than the timing of the minus maximum turning amount. For example, the "leftward trunk turning amount" is the absolute value of the difference between the minus maximum turning amount at the timing of e2 and the plus maximum turning amount at the timing of e3.

Meanwhile, regarding the successive plus and minus maximum turning amounts, the trunk turning amount calculation unit 37B calculates an absolute value of a difference between the plus and minus maximum turning amounts as a "rightward trunk turning amount" when the timing of the plus maximum turning amount comes earlier than the timing of the minus maximum turning amount. For example, the "rightward trunk turning amount" is the absolute value of the difference between the minus maximum turning amount at the timing of e2 and the plus maximum turning among at the timing of e1.

### [A specific example of right-and-left turning amount calculation]

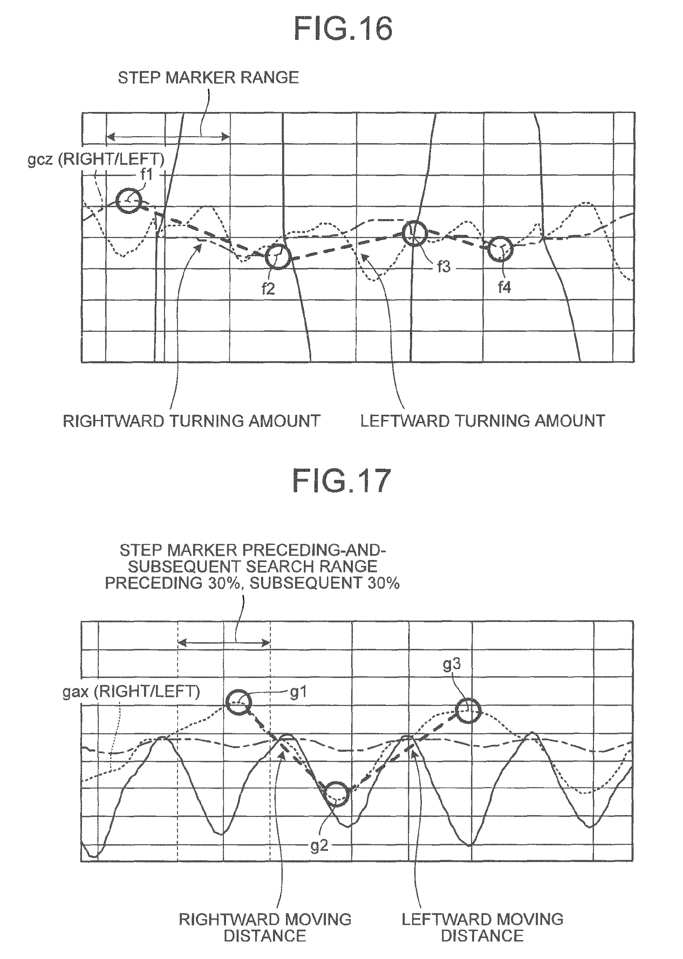

Next, a specific example of right-and-left turning amount calculation by the right-and-left turning amount calculation unit 37C will be described with reference to FIG. 16. FIG. 16 is an explanation diagram describing a specific example of the right-and-left turning amount calculation. The right-and-left turning amount calculation by the right-and-left turning amount calculation unit 37C uses the rightward and leftward turning amounts gcz from among the various turning amounts and moving distances. Among the rightward and leftward turning amounts gcz, a rightward turning amount indicates plus and a leftward turning amount indicates minus. Note that a reference of the rightward and leftward turning amounts is, for example, an attached posture at a start timing of a walking posture diagnosis of a mobile phone to which a six-axis sensor 24 is equipped.

The right-and-left turning amount calculation unit 37C selects successive step markers and calculates a range between the selected two step markers as a step marker range. Then, the right-and-left turning amount calculation unit 37C specifies timings of becoming right and left maximum turning amounts from the step marker range. That is, the right-and-left turning amount calculation unit 37C specifies a plus maximum turning amount and a minus maximum turning amount from the step marker range. Here, the timings of f1 and f3 are specified as the timings of the plus maximum turning amounts and the timings of f2 and f4 are specified as the timings of the minus maximum turning amounts. Then, regarding successive plus and minus maximum turning amounts, the right-and-left turning amount calculation unit 37C calculates an absolute value of a difference between the plus and minus maximum turning amounts as a "leftward turning amount" when the timing of the plus maximum turning amount comes later than the timing of the minus maximum turning amount. For example, the "leftward turning amount" is the absolute value of the difference between the minus maximum turning amount at the timing of f2 and the plus maximum turning amount at the timing of f3.

Meanwhile, regarding the successive plus and minus maximum turning amounts, the right-and-left turning amount calculation unit 37C calculates an absolute value of a difference between the plus and minus maximum turning amounts as a "rightward turning amount" when the timing of the plus maximum turning amount comes earlier than the timing of the minus maximum turning amount. For example, the "rightward turning amount" is the absolute amount of the difference between the minus maximum turning amount at the timing of f2 and the plus maximum turning amount at the timing of f1.

### [A specific example of right-and-left moving distance calculation]

Next, a specific example of right-and-left moving distance calculation by the right-and-left moving distance calculation unit 37D will be described with reference to FIG. 17. FIG. 17 is an explanation diagram describing a specific example of the right-and-left moving distance calculation. The right-and-left moving distance calculation by the right-and-left moving distance calculation unit 37D uses the rightward and leftward moving distances gax from among the various turning amounts and moving distances. Among the rightward and leftward moving distances gax, a leftward moving distance indicates plus and a rightward moving distance indicates minus. Note that a reference of the rightward and leftward moving distances is, for example, an attached posture at a start timing of a walking posture diagnosis of a mobile phone to which a six-axis sensor 24 is equipped. Also, a method of calculating the preceding-and-subsequent search range is similar to the step marker right-and-left determination unit 36, and therefore description thereof is omitted.

The right-and-left moving distance calculation unit 37D specifies respective turning-back points of right and left moving distances from the preceding-and-subsequent search range after the calculation of the preceding-and-subsequent search range. That is, the right-and-left moving distance calculation unit 37D specifies a plus maximum moving distance and a minus maximum moving distance from the preceding-and-subsequent search range. Here, the timings of g1 and g3 are specified as the timings of the plus maximum moving distance and the timing of g2 is specified as the timing of the minus maximum moving distance. Then, regarding successive plus and minus maximum moving distances, the right-and-left moving distance calculation unit 37D calculates an absolute value of a difference between the plus and minus maximum moving distances as a "leftward moving distance" when the timing of the plus maximum moving distance comes later than the timing of the minus maximum moving distance. For example, the "leftward moving distance" is the absolute value of the difference between the minus maximum moving distance at the timing of g2 and the plus maximum moving distance at the timing of g3.

Meanwhile, regarding the successive plus and minus maximum moving distances, the right-and-left moving distance calculation unit 37D calculates an absolute value of a difference between the plus and minus maximum turning amounts as a "rightward moving distance" when the timing of the plus maximum moving distance comes earlier than the timing of the minus maximum moving distance. For example, the "right direction moving distance" is the absolute value of the difference between the minus maximum moving distance at the timing of g2 and the plus maximum moving distance at the timing of g1

### [A specific example of up-and-down moving distance calculation]

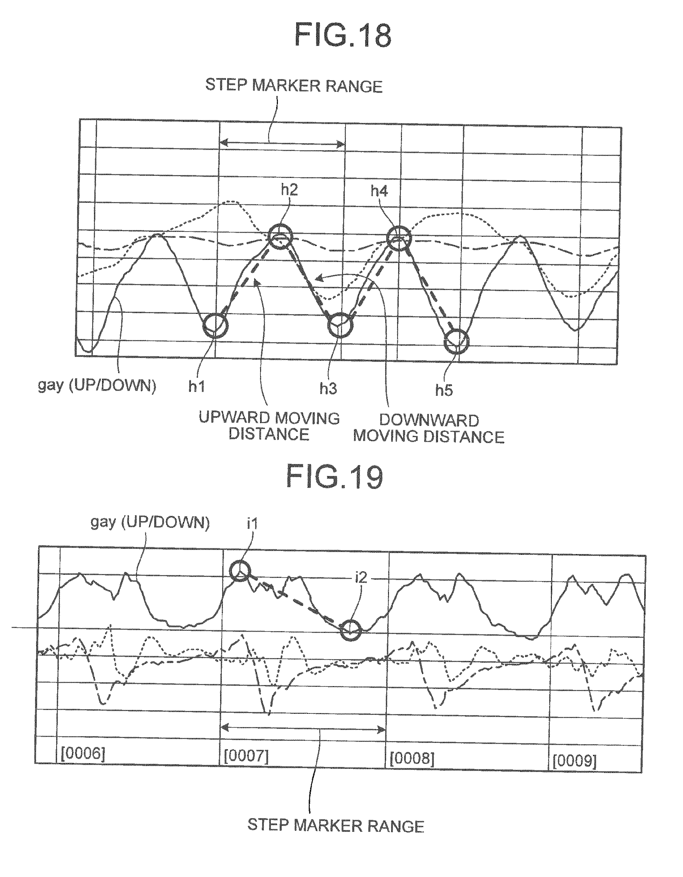

Next, a specific example of up-and-down moving distance calculation by the up-and-down moving distance calculation unit 37E will be described with reference to FIG. 18. FIG. 18 is an explanation diagram describing a specific example of the up-and-down moving distance calculation. The up-and-down moving distance calculation by the up-and-down moving distance calculation unit 37E uses the upward and downward moving distances gay from among the various turning amounts and moving distances. Among the upward and downward moving distances gay, an upward moving distance indicates plus and a downward moving distance indicates minus. Note that a reference of the upward and downward moving distances is, for example, an attached posture at a start timing of a walking posture diagnosis of a mobile phone to which a six-axis sensor 24 is equipped.

The up-and-down moving distance calculation unit 37E selects successive step markers and calculates a range between the selected two step markers as the step marker range. Then, the up-and-down moving distance calculation unit 37E specifies an already extracted step marker in order to specify the timing of becoming a minus maximum moving distance. Here, it is the timings h1, h3, and h5.

Then, the up-and-down moving distance calculation unit 37E specifies a plus maximum moving distance from the step marker range. Here, it is the timings of h2 and h4. Then, regarding successive plus and minus maximum moving distances, the up-and-down moving distance calculation unit 37E calculates an absolute value of a difference between the plus and minus maximum moving distances as an "upward moving distance" when the timing of the minus maximum moving distance comes earlier than the timing of the plus maximum moving distance. For example, the "upward moving distance" is the absolute value of the difference between the minus maximum moving distance at the timing of h1 and the plus maximum moving distance at the timing of h2.

Meanwhile, regarding the successive plus and minus maximum moving distances, the up-and-down moving distance calculation unit 37E calculates an absolute value of a difference between the plus and minus maximum moving distances as a "downward moving distance" when the timing of the minus maximum moving distance comes later than the timing of the plus maximum moving distance. For example, the "downward moving distance" is the absolute value of the difference between the plus maximum moving distance at the timing of h2 and the minus maximum moving distance at the timing of h3.

### [A specific example of landing impact calculation]

Next, a specific example of landing impact calculation by the landing impact calculation unit 37F will be described with reference to FIG. 19. FIG. 19 is an explanation diagram describing a specific example of the landing impact calculation. The landing impact calculation by the landing impact calculation unit 37F uses the upward and downward moving distances gay from among the various turning amounts and moving distances. Among the upward and downward moving distances gay, an upward moving distance indicates plus and a downward moving distance indicates minus. Note that a reference of the upward and downward moving distances is, for example, an attached posture at a start timing of a walking posture diagnosis of a mobile phone to which a six-axis sensor 24 is equipped.

The landing impact calculation unit 37F calculates successive step markers and calculates a range between the selected two step markers as the step marker range. Then, the landing impact calculation unit 37F specifies upward and downward maximum moving distances from the step marker range. For example, the timing of i1 in the step marker range is specified as an upward maximum moving distance and the timing of i2 in the step marker range is specified as a downward maximum moving distance. Then, the landing impact calculation unit 37F calculates an absolute value of a difference between the successive upward and downward maximum moving distances as an "impact of landing". For example, the "impact of landing" is the absolute value of the difference between the upward maximum moving distance at the timing of i1 and the downward maximum moving distance at the timing of i2.

### [A procedure of a walking posture diagnostic process according to the embodiment 2]

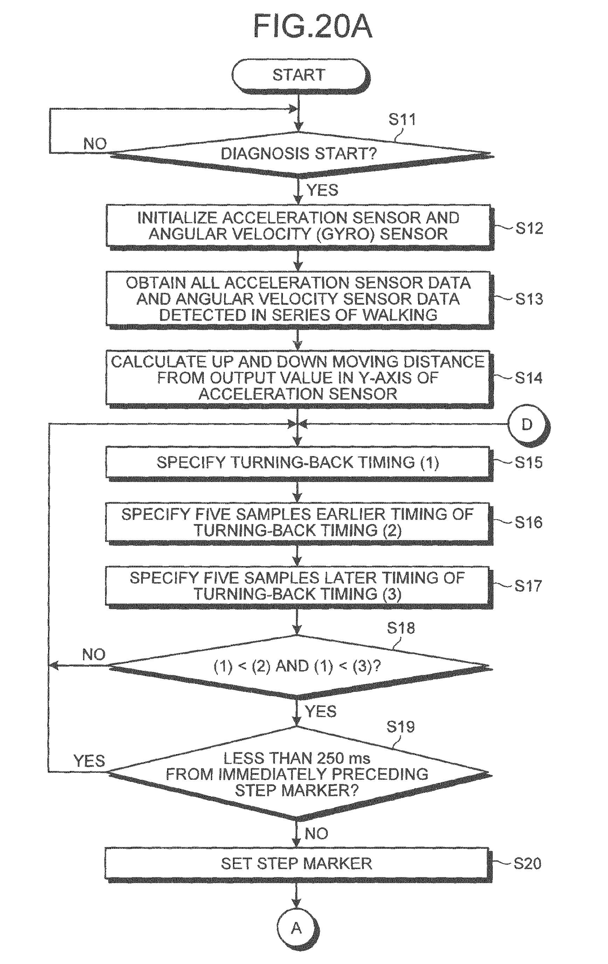

Next, a procedure of a walking posture diagnostic process according to the embodiment 2 will be described with reference to FIGS. 20A to 20D. FIG. 20A is a flowchart illustrating a procedure of a step marker extraction process according to the embodiment 2.

First, the input control unit 33 determines whether there is a start instruction of a walking posture diagnosis (step S11). Then, when determining that there is no start instruction of a walking posture diagnosis (No at step S11), the process transfers to step S11 for waiting until an instruction. Meanwhile, when determining that there is a start instruction of a walking posture diagnosis (Yes at step S11), the six-axis sensor control unit 34 initializes the six-axis sensor 24 (step S12). That is, the six-axis sensor control unit 34 initializes the acceleration sensor and the angular velocity (gyro) sensor that constitute the six-axis sensor 24.

Then, the six-axis sensor control unit 34 obtains all of various types of data detected by the acceleration sensor and the angular velocity sensor in a series of walking based on the start instruction of a walking posture diagnosis (step S13). For example, the six-axis sensor control unit 34 obtains the various types of data of one hour from the six-axis sensor 24 by 60 samples per a second. Then, the six-axis sensor control unit 34 calculates an up-and-down moving distances from an output value of the y-axis of the acceleration sensor that constitutes the six-axis sensor 24 (step S14). Note that the six-axis sensor control unit 34 may include, as the various types of data from the six-axis sensor 24, data of the calculated up-and-down moving distances, right-and-left moving distances, front-back moving distances, front-back turning amounts, trunk turning amount, and right-and-left turning amounts. Then, the six-axis sensor control unit 34 stores these various types of data in the data storage unit 44 as raw data.

Then, the step marker extraction unit 35 refers to the up-and-down moving distances at a plurality of timings and specifies a downward (minus) turning-back timing (step S15). Then, the step marker extraction unit 35 specifies the timing of five samples earlier than the specified turning-back timing (step S16) while specifying the timing of five samples later than the specified turning-back timing (step S17).

Next, the step marker extraction unit 35 determines whether the moving distance at the specified turning-back timing is smaller than the moving distance at the five-sample-earlier timing and is smaller than the moving distance at the five-sample-later timing (step S18). Then, when the movement distance at the turning-back timing is the moving distance at the five-sample-earlier timing or more, or is the moving distance at the five-samples-later timing or more (No at step S18), the step marker extraction unit 35 determines that the turning-back timing is not a step marker and transfers to step S15.

Meanwhile, when the moving distance at the turning-back timing is smaller than the moving distance at the five-sample-earlier timing, and is smaller than the moving distance at the five-sample-later timing (Yes at step S18), the step marker extraction unit 35 extracts the turning-back timing as a step marker.

Further, the step marker extraction unit 35 determines whether the extracted step marker is less than 250 ms from an immediately preceding step marker (step S19). Then, when the extracted step marker is less than 250 ms from the immediately preceding step marker (Yes at step S19), the step marker extraction unit 35 determines that the extracted step marker is not a step of the walking posture and is not a step marker. Then, the step marker extraction unit 35 transfers to step S15.

Meanwhile, when the extracted step marker is not less than 250 ms from the immediately preceding step marker (No at step S19), the step marker extraction unit 35 sets a distinction as a step marker in the raw data (step S20) and transfers to a step marker right-and-left determination process.

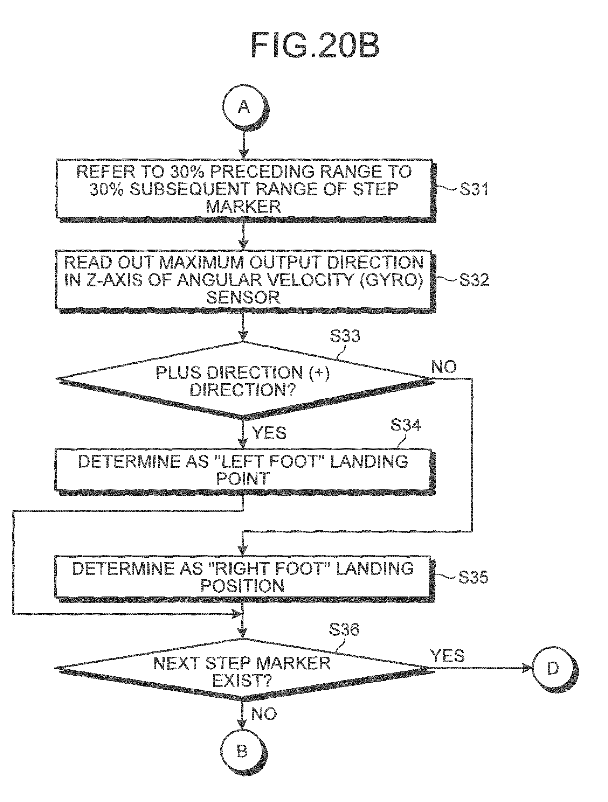

Next, a procedure of the step marker right-and-left determination process will be described with reference to FIG. 20B. FIG. 20B is a flowchart illustrating a procedure of the step marker right-and-left determination process.

First, the step marker right-and-left determination unit 36 refers to a 30% preceding range to a 30% subsequent range of the step marker in the preceding-and-subsequent search range in the raw data (step S31), and reads out a maximum output direction in the z-axis of the angular velocity (gyro) sensor from the preceding-and-subsequent search range (step S32). That is, the step marker right-and-left determination unit 36 reads out a maximum turning amount in the right-and-left direction from the preceding-and-subsequent search range.

Next, the step marker right-and-left determination unit 36 determines whether the read out maximum output direction is in the plus direction (step S33). Then, when the maximum output direction is in the plus direction (Yes at step S33), the step marker right-and-left determination unit 36 determines that it is a landing point of a "left foot" because the turning is in the right direction (step S34). Meanwhile, when the maximum output direction is in the minus direction (No at step S33), the step marker right-and-left determination unit 36 determines that is a landing position of a "right foot" because the turning is in the left turning (step S35).

Next, the step marker right-and-left determination unit 36 determines whether a next step marker exists (step S36). Then, when the next step marker exists (Yes at step S36), the process transfers to step S15. Meanwhile, when no next step marker exists (No at step S36), the step marker right-and-left determination unit 36 adds a distinction of a right/left foot that constitutes a step to data of the step marker in the raw data, and outputs the added data to the walking posture locus amount calculation unit 37. Then, the step marker right-and-left determination unit 36 transfers to a walking posture locus amount calculation process.

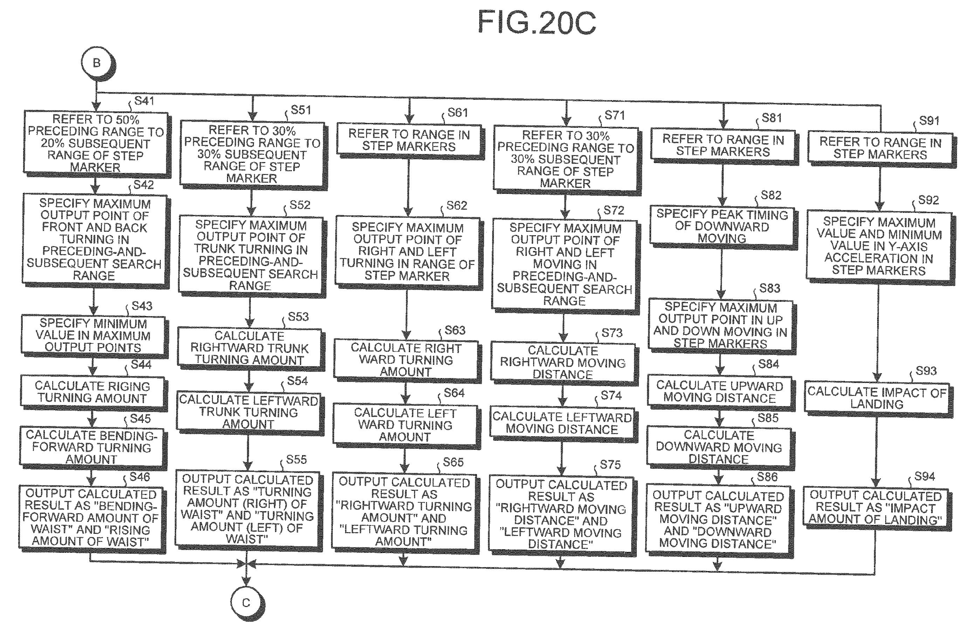

Next, a procedure of the walking posture locus amount calculation process will be described with reference to FIG. 20C. FIG. 20C is a flowchart illustrating a procedure of the walking posture locus amount calculation process.

First, the front-back turning amount calculation unit 37A refers to the preceding-and-subsequent search range of 50% preceding range and 20% subsequent range of the step marker from the output data output by the step marker right-and-left determination unit 36 (step S41). Then, the front-back turning amount calculation unit 37A specifies a maximum output point in the x-axis of the angular velocity (gyro) sensor from the preceding-and-subsequent search range (step S42). That is, the front-back turning amount calculation unit 37A specifies, from the preceding-and-subsequent search range, a backward maximum turning amount (in the rising direction) at which the turning amount in the front-and-back direction becomes plus.

Next, the front-back turning amount calculation unit 37A specifies a minimum value in successive maximum output points (step S43). That is, the front-back turning amount calculation unit 37A specifies a forward maximum turning amount (in the bending-forward direction) in a period having successive maximum turning amounts among the specified backward maximum turning amounts. Then, the front-back turning amount calculation unit 37A calculates an absolute value of a difference between the turning amounts as a "rising turning amount" when the timing of the maximum output point comes later than the timing of the minimum value (step S44).

Then, the front-back turning amount calculation unit 37A calculates an absolute value of a difference between the turning amounts as a "bending-forward turning amounts" when the timing of the maximum output point comes earlier than the timing of the minimum value (step S45). Then, the front-back turning amount calculation unit 37A outputs the calculated "rising turning amount" as a "rising amount of a waist" and the calculated "bending-forward turning amount" as a "bending-forward amount of a waist" (step S46).

Next, the trunk turning amount calculation unit 37B refers to a 30% preceding range to a 30% subsequent range of the step marker in the preceding-and-subsequent search range from the output data output from the step marker right-and-left determination unit 36 (step S51). Then, the trunk turning amount calculation unit 37B specifies maximum output points in the y-axis of the angular velocity (gyro) sensor from the preceding-and-subsequent search range (step S52). That is, the trunk turning amount calculation unit 37B specifies rightward and leftward maximum turning amounts of the trunk turning from the preceding-and-subsequent search range.

Next, regarding successive rightward and leftward maximum turning amounts, the trunk turning amount calculation unit 37B calculates an absolute value of a difference between the rightward and leftward turning amounts as a "rightward trunk turning amount" when the timing of the rightward maximum turning amount comes earlier than the timing of the leftward maximum turning amount (step S53). Then, regarding the successive rightward and leftward maximum turning amounts, the trunk turning amount calculation unit 37B calculates an absolute value of a difference between the rightward and leftward turning amounts as a "leftward trunk turning amount" when the timing of the rightward maximum turning amount comes later than the timing of the leftward maximum turning amount (step S54).

Then, the trunk turning amount calculation unit 37B outputs the calculated "rightward trunk turning amount" as a "turning amount (right) of a waist" and the calculated "leftward trunk turning amount" as a "turning amount (left) of a waist" (step S55).

Next, the right-and-left turning amount calculation unit 37C refers to the step marker range between the step markers from the output data output from the step marker right-and-left determination unit 36 (step S61). Then, the right-and-left turning amount calculation unit 37C specifies maximum output points of the right and left turning in the step marker range (step S62). That is, the right-and-left turning amount calculation unit 37C specifies maximum turning amounts in the right-and-left direction from the step marker range.

Next, regarding successive rightward and leftward maximum turning amounts, the right-and-left turning amount calculation unit 37C calculates an absolute value of a difference between the rightward and leftward turning amounts as a "leftward turning amount" when the timing of the leftward maximum turning amount comes earlier than the timing of the rightward maximum turning amount (step S63). Then, regarding the successive rightward and leftward maximum turning amounts, the right-and-left turning amount calculation unit 37C calculates an absolute value of a difference between the rightward and leftward turning amounts as a "rightward turning amount" when the timing of the leftward maximum turning amount comes later than the timing of the rightward maximum turning amount (step S64).

Then, the right-and-left turning amount calculation unit 37C outputs the calculated "rightward turning amount" as a "turning amount in the right direction" and the calculated "leftward turning amount" as a "turning amount in the left direction" (step S65).

Next, the right-and-left moving distance calculation unit 37D refers to a 30% preceding range to a 30% subsequent range of the step marker in the preceding-and-subsequent search range from the output data output from the step marker right-and-left determination unit 36 (step S71). Then, the right-and-left moving distance calculation unit 37D specifies maximum output points of the right and left movement in the preceding-and-subsequent search range (step S72). That is, the right-and-left moving distance calculation unit 37D specifies maximum moving distances in the right-and-left direction from the preceding-and-subsequent search range.

Next, regarding successive rightward and leftward maximum moving distances, the right-and-left moving distance calculation unit 37D calculates an absolute value of a difference between the rightward and leftward moving distances as a "rightward moving distance" when the timing of the leftward maximum moving distance comes earlier than the timing of the rightward maximum moving distance (step S73). Then, regarding the successive rightward and leftward maximum moving distances, the right-and-left moving distance calculation unit 37D calculates an absolute value of a difference between the rightward and leftward moving distances as a "leftward moving distance" when the timing of the leftward maximum moving distance comes later than the timing of the rightward maximum moving distance (step S74).

Then, the right-and-left moving distance calculation unit 37D outputs the calculated "rightward moving distance" as a "moving distance in the right direction" and the calculated "leftward moving distance" as a "moving distance in the left direction" (step S75).

Next, the up-and-down moving distance calculation unit 37E refers to the step marker range between step markers from the output data output from the step marker right-and-left determination unit 36 (step S81). Then, the up-and-down moving distance calculation unit 37E specifies peak timings of a downward movement in the step marker range (step S82). That is, the up-and-down moving distance calculation unit 37E specifies step markers at both ends of the step marker range.

Then, the up-and-down moving distance calculation unit 37E specifies upward and downward maximum output points between step markers (step S83). That is, the up-and-down moving distance calculation unit 37E specifies an upward maximum moving distance from the step marker range.

Next, regarding successive upward and downward maximum moving distances, the up-and-down moving distance calculation unit 37E calculates an absolute value of a difference between the upward and downward moving distances as an "upward moving distances" when the timing of the downward maximum moving distance comes earlier than the timing of the upward maximum moving distance (step S84). Then, regarding the successive upward and downward maximum moving distances, the up-and-down moving distance calculation unit 37E calculates an absolute value of a difference between the upward and downward moving distances as a "downward moving distance" when the timing of the downward maximum moving distance comes later than the timing of the upward maximum moving distance (step S85).

Then, the up-and-down moving distance calculation unit 37E outputs the calculated "upward moving distance" as a "moving distance in the upward direction" and the calculated "downward moving distance." as a "moving distance in the downward direction" (step S86).

Next, the landing impact calculation unit 37F refers to the step marker range between step markers from the output data output from the step marker right-and-left determination unit 36 (step S91). The landing impact calculation unit 37F specifies a maximum value and a minimum value in the y-axis of the acceleration sensor from the step marker range (step S92). That is, the landing impact calculation unit 37F specifies upward and downward maximum moving distances from the step marker range.

Next, the landing impact calculation unit 37F calculates an absolute value of a difference between the upward and downward maximum moving distances as an "impact of landing" (step S93). Then, the landing impact calculation unit 37F outputs the calculated "impact of landing" as an "impact amount of landing" (step S94).

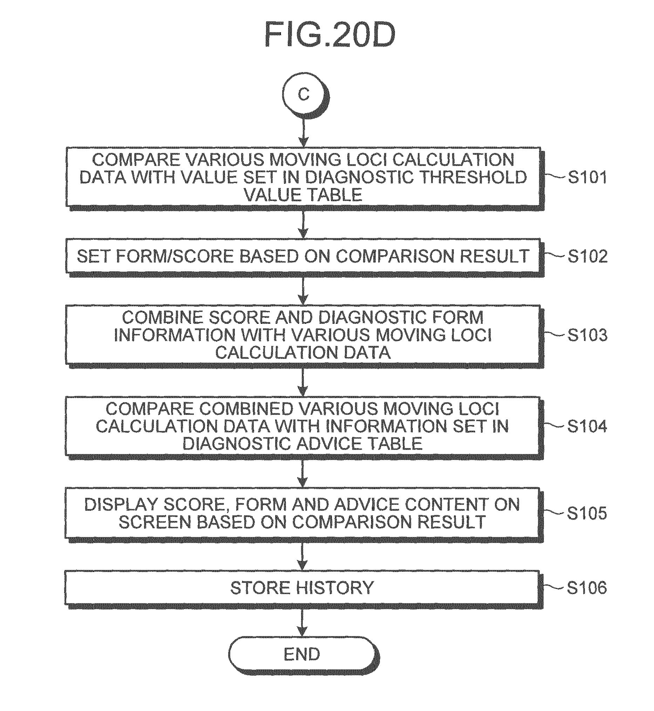

Next, a procedure of a walking posture diagnostic process will be described with reference to FIG. 20D. FIG. 20D is a flowchart illustrating a procedure of the walking posture diagnostic process according to the embodiment 2.

First, the walking posture diagnostic unit 38 compares "calculation data of various moving loci" with a value set in the diagnostic threshold value table 41 (step S101). The "calculation data of various moving loci" is data in which an average value is calculated for each moving locus using data of the various moving loci output from the walking posture locus amount calculation unit 37.

Then, the walking posture diagnostic unit 38 calculates a score according to a predetermined scoring method based on a comparison result, and creates diagnostic form (walking posture) information (step S102). Then, the walking posture diagnostic unit 38 combines the "calculation data of various moving loci" with the diagnostic form (walking posture) information and the score (step S103).

Next, the display control unit 39 compares the combined "calculation data of various moving loci" with information set in the diagnostic advice table 42 (step S104). Then, the display control unit 39 edits the "calculation data of various moving loci" from the comparison result and displays the score, the diagnostic form (walking posture) information, and an advice content on a screen (step S105).

Then, the display control unit 39 stores the "calculation data of various moving loci" and the advice content in the diagnostic history table 43 (step S106).

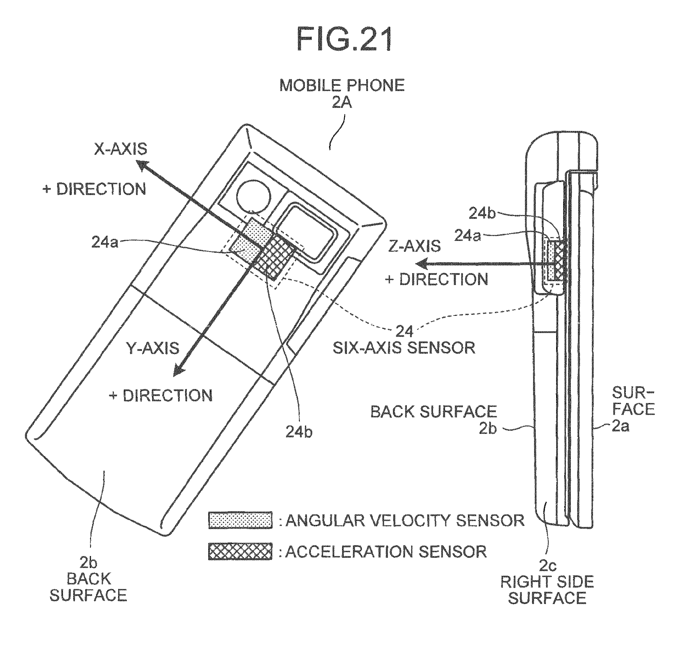

Next, an equipped position of the six-axis sensor 24 when the mobile terminal device 2 serves as a mobile phone will be described with reference to FIG. 21. FIG. 21 is a diagram illustrating an example of an equipped position of the six-axis sensor 24 when the mobile terminal device 2 serves as a mobile phone. As illustrated in FIG. 21, the six-axis sensor 24 including the angular velocity sensor 24a and the acceleration sensor 24b is equipped in a right side surface 2c and in a back surface 2b in reference to a surface 2a of a mobile phone 2A. Also, the six-taxis sensor 24 in the back surface 2b of the mobile phone 2A is provided with the y-axis, one of longer direction indicating plus, and is provided with the x-axis, one of shorter direction indicating minus. The six-axis sensor 24 in the right side surface 2c of the mobile phone 2A is provided with the z-axis, the vertical direction from the back surface 2b indicating plus.

Note that, when a diagnosis of a walking posture is performed, the mobile phone 2A is attached to the subject, and it is desirable to attach the mobile phone 2A in such a way that the coordinate-axis of the six-axis sensor 24 and the reference coordinate axis coincide with each other. However, even if the coordinate-axis of the six-axis sensor 24 is turned in accordance with the attached posture of the mobile phone 2A, the acceleration sensor 24b included in the six-axis sensor 24 is capable of determining a maximum direction of gravitational acceleration. Therefore, if the coordinate-axis in accordance with the attached posture of the mobile phone 2A is turned so as to coincide with the reference coordinate axis using the maximum direction of the gravitational acceleration, data corresponding to the reference coordinate axis can be detected. Therefore, the attached posture of the mobile phone 2A can employ any posture.

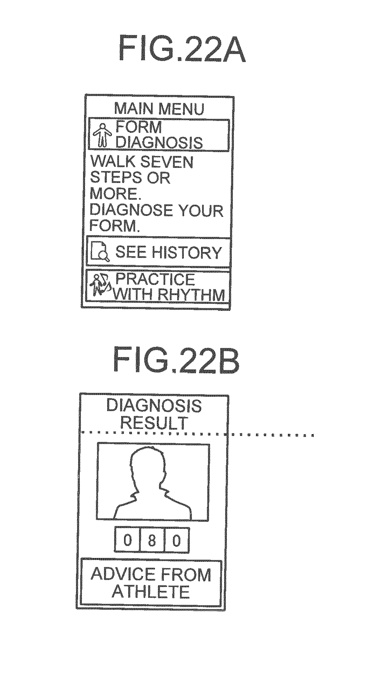

Next, an example of a display screen displayed by the display unit 23 will be described with reference to FIGS. 22A to 22G. FIGS. 22A to 22G are diagrams illustrating an example of a display screen. As illustrated in FIG. 22A, the display unit 23 displays a main menu in relation to a diagnosis of a walking posture. For example, the main menu includes menus such as "form diagnosis" and "See history'", and the menus are selected by a cursor key of the input unit 22. Here, when "Form diagnosis" is selected by the input unit 22, a start instruction of a walking posture diagnosis is output to the input control unit 33.

As illustrated in FIG. 22B, the display unit 23 displays a diagnosis result screen of the walking posture. For example, the diagnosis result screen of the walking posture includes a menu of "Advice from an athlete" and this menu is selected by the cursor key of the input unit 22.

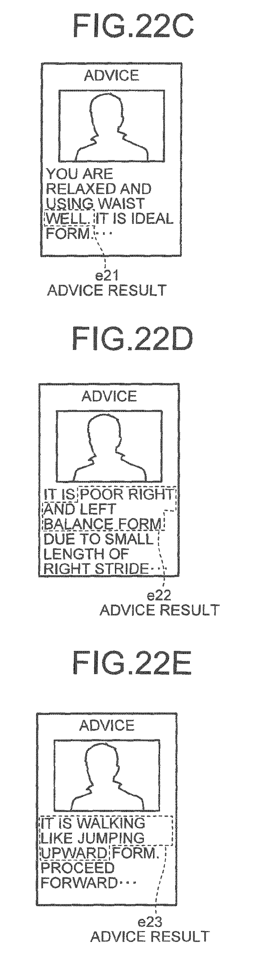

As illustrated in FIGS. 22C to 22E, the display unit 23 displays an advice content obtained in such a way that the "calculation data of various moving loci" is edited by the display control unit 39. FIG. 22C is a display example of the advice content when the various moving loci satisfy a reference. For example, the display unit 23 displays it is an "ideal" form. FIG. 22D is a display example of the advice content when a rightward turning amount or a leftward turning amount does not satisfy the reference. For example, the display unit 23 displays it is a "poor right and left balance" form. FIG. 22E is a display example of the advice content when an upward moving distance does not satisfy the reference. For example, the display unit 23 displays it is a "walking like jumping upward" form. Note that "ideal" e21, "poor right and left balance" e22, and "walking like jumping upward" e23 are set as advice results e2 of the "calculation data of various moving loci".

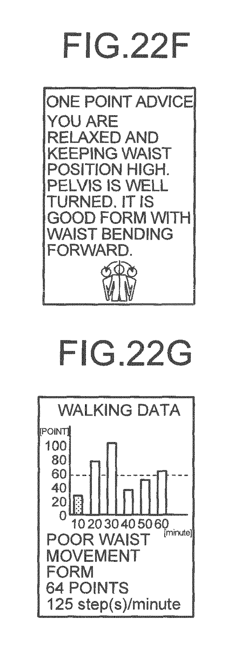

As illustrated in FIG. 22F, the display unit 23 displays one point advice content based on the above-described advice content. For example, the one point advice content is a content set in a message content 42c in the diagnostic advice table 42.

As illustrated in FIG. 22G, the display unit 23 displays walking data. That is, when "See history" is selected by the input unit 22, the walking data obtained based on a content stored in the diagnostic history table 43 and the data storage unit 44 is displayed. For example, the diagnostic form (walking posture) information, the score, and the like set in the "calculation data of various moving loci" are displayed.

### [Effects of the embodiment 2]

According to the above-described embodiment 2, the step marker extraction unit 35 selects a downward turning-back timing from among upward and downward moving distances at a plurality of timings. Then, the step marker extraction unit 35 extracts the selected timing as a step marker when the moving distance at the selected timing is smaller than the moving distance at five-sample-earlier timing, and the moving distance at the selected timing is smaller than the moving distance at five-sample-later timing. According to such a configuration, the step marker extraction unit 35 is capable of excluding a case of a turning back at the turning-back timing that indicates small fluctuation, whereby a step marker that appears at every step can be accurately extracted. As a result, the step marker extraction unit 35 is capable of accurately obtaining a moving distance in relation to each step of successive step markers by walking, and is capable of efficiently calculating a moving locus of a walking posture caused by the walking based on each of the obtained moving distances.

Further, according to the above-described embodiment 2, the step marker extraction unit 35 does not take an extracted step marker as a step marker when the extracted step marker falls outside a reference period that is taken for a step from an immediately preceding step marker. According to such a configuration, the step marker extraction unit 35 is capable of accurately extracting a step marker that appears at every step by not taking the step marker falling outside the reference period that is taken for a step as a step marker. As a result, the step marker extraction unit 35 is capable of further accurately obtaining a moving distance in relation to a step of the successive step markers by walking, and is capable of efficiently calculating a moving locus of a walking posture caused by the walking based on each of the obtained moving distances.

Further, according to the above-described embodiment 2, the right-and-left turning amount calculation unit 37C obtains turning amounts associated with turning in the right-and-left direction in walking of a subject detected by the six-axis sensor 24. Then, the right-and-left turning amount calculation unit 37C calculates a difference between a maximum turning amount caused by an immediately preceding step marker of an extracted step marker and a maximum turning value caused by the extracted step marker as a "right and left turning locus". According to such a configuration, the right-and-left turning amount calculation unit 37C calculates the right and left turning locus from the maximum turning amounts caused by successive step markers. Therefore, the right and left turning balance based on the calculated right and left turning locus can be efficiently diagnosed.

Further, according to the above-described embodiment 2, the step marker right-and-left determination unit 36 obtains turning amounts associated with turning in the right-and-left direction in walking by the subject detected by the six-axis sensor 24. Then, the step marker right-and-left determination unit 36 determines a right/left foot that constitutes a step based on the plus/minus sign of an immediately subsequent maximum turning amount of an extracted step marker. According to such a configuration, the step marker right-and-left determination unit 36 determines the right/left foot that constitutes a step based on the plus/minus sign of the immediately subsequent maximum turning amount of the step. Therefore, the right/left foot that constitutes a step of the step marker can be easily determined.

Further, according to the above-described embodiment 2, the mobile terminal device 2 includes the six-axis sensor 24. According to such a configuration, the mobile terminal device 2 is capable of detecting various types of data in relation to walking, whereby, various moving loci in relation to the walking can be calculated and some advice in relation to a walking posture can be provided based on a plurality of moving loci.

Further, according to the above-described embodiment 2, the display control unit 39 displays a diagnosis result diagnosed by the walking posture diagnostic unit 38 on the display unit 23. According to such a configuration, the display control unit 39 is capable of notifying the subject who had the diagnosis of the diagnosis result, and is capable of efficiently providing advice in relation to the walking posture.

### [A program and the like]

Note that the step marker extraction unit 35 determines a step of the walking posture using the upper and lower limits. That is, the step marker extraction unit 35 determines whether the step marker is less than 250 ms that indicates the lower limit from the immediately preceding step marker. Also, the step marker extraction unit 35 determines whether the step marker exceeds 1000 ms that indicates the upper limit from the immediately preceding step marker. However, these upper and lower limits are not limited to 250 ms and 1000 ms and may be changed. Consequently, the mobile terminal device 2 becomes capable of changing the limits into a value according to a walking timing of the subject, so that the locus of the walking posture in accordance with the walking of the subject can be properly calculated.

Also, the step marker extraction unit 35 extracts a characteristic timing that appears at every step as a step marker based on the upward and downward moving distances. In the embodiment, this characteristic timing has been described as a landing timing of a step. However, the characteristic timing is not limited to the landing time and, for example, it may be a timing when a foot lifts at a maximum.

Further, the mobile terminal device 2 includes the six-axis sensor 24. That is, the six-axis sensor control unit 34 detects the rightward and leftward moving distances, the upward and downward moving distances, the forward and backward moving distances, the forward and backward turning amounts, the turning amount in the trunk direction, and the rightward and leftward turning amounts, output from the six-axis sensor 24. However, the mobile terminal device 2 is not limited to the above example, and may includes an acceleration sensor in the y-axis direction that detects the upward and downward moving distances among the triaxial acceleration sensor and an acceleration sensor of one of the coordinate axes other than the y-axis or an angular velocity sensor around one of the coordinate axes.

Further, each configuration element of each illustrated mobile terminal device is not necessarily physically configured as illustrated in the drawings. That is, a specific aspect of dispersion/integration of each device is not limited to the illustrated examples, and the whole or a part thereof can be configured by being dispersed/integrated according to various loads, a use condition, and the like. For example, the walking posture diagnostic unit 38 and the display control unit 39 may be integrated as one unit. Meanwhile, the step marker extraction unit 35 can be divided into the extraction unit and a step marker determination unit that determines whether the turning-back timing is a step marker. Also, the storage unit 26 may be connected as an external device of the mobile terminal device 2 via a network.

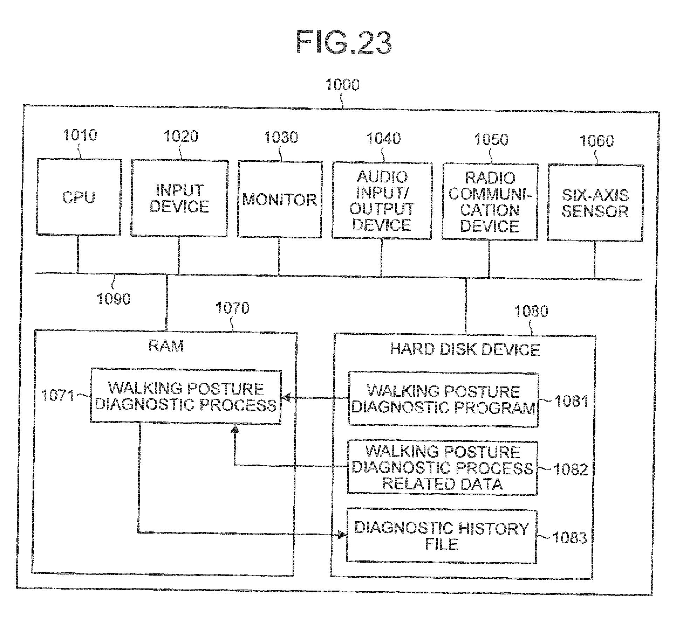

Also, the various processes described in the above embodiment can be realized by causing a computer to execute a program prepared in advance with. Therefore, hereinafter, an example of a computer that executes a walking locus calculation program having a similar function to the mobile terminal device 2 illustrated in FIG. 2 will be described with reference to FIG. 23.

FIG. 23 is a diagram illustrating a computer that executes a walking locus calculation program. As illustrated in FIG. 23, a computer 1000 includes a central processing unit (CPU) 1010, an input device 1020, a monitor 1030, an audio input/output device 1040, a radio communication device 1050, and a six-axis sensor 1060. Further, the computer 1000 includes a RAM 1070 and a data storage device such as a hard disk device 1080, and these devices are connected by a bus 1090. The CPU 1010 executes various arithmetic processes. The input device 1020 receives an input of data from a user. The monitor 1030 displays various types of information. The audio input/output device 1040 inputs/outputs audio. The radio communication device 1050 performs transmission/reception of data with other computer via radio communication. The six-axis sensor 1060 detects acceleration in the three axis directions and angular velocity around the three axes. The RAM 1070 temporarily stores various types of information.

Then, a walking posture diagnostic program 1081 that has a similar function to the control unit 25 illustrated in FIG. 2 is stored in the hard disk device 1080. Also, a walking posture diagnostic process related data 1082 corresponding to the various types of data (diagnostic threshold value table 41, the diagnostic advice table 42, the diagnostic history table 43) that are stored in the storage unit 26 illustrated in FIG. 2 and a diagnostic history file 1083 are stored in the hard disk device 1080.

Then, the CPU 1010 reads out the walking posture diagnostic program 1081 from the hard disk device 1080 and expands it in the RAM 1070, so that the walking posture diagnostic program 1081 functions as a walking posture diagnostic process 1071. Then, the walking posture diagnostic process 1071 properly expands information and the like read out from the walking posture diagnostic process related data 1082 in a region allocated to itself in the RAM 1070 and executes various data processes based on this expanded data and the like. Then, the walking posture diagnostic process 1071 outputs predetermined information to the diagnostic history file 1083.

Note that the above-described walking posture diagnostic program 1081 is not necessarily stored in the hard disk device 1080, and this program stored in a storage medium such as a CD-ROM may be read out and executed by the computer 1000. Also, this program may be stored in other computer (or a server) connected to the computer 1000 through a public line, the internet, a local area network (LAN), a wide area network (WAN), and the like. In this case, the computer 1000 reads out and executes these programs therefrom.

### Reference Signs List

### [Explanation of Reference]

- 1 and 2: Mobile terminal device
- 11: First displacement detection unit
- 12: Second displacement detection unit
- 13: Walking posture reference locus storage unit
- 14: Characteristic displacement timing extraction unit
- 15: Locus calculation unit
- 16: Walking posture determination unit
- 21: Radio communication unit
- 22: Input unit
- 23: Display unit
- 24: Six-axis sensor
- 25: Control unit
- 26: Storage unit
- 31: Radio control unit
- 32: Call control unit
- 33: Input control unit
- 34: Six-axis sensor control unit
- 35: Step marker extraction unit
- 36: Step marker right-and-left determination unit
- 37: Walking posture locus amount calculation unit
- 37A: Front-back turning amount calculation unit
- 37B: Trunk turning amount calculation unit
- 37C: Right-and-left turning amount calculation unit
- 37D: Right-and-left moving distance calculation unit
- 37E: Up-and-down moving distance calculation unit
- 37F: Landing impact calculation unit
- 38: Walking posture diagnostic unit
- 39: Display control unit
- 41: Diagnostic threshold value table
- 42: Diagnostic advice table
- 43: Diagnostic history table
- 44: Data storage unit

## Claims

1. A mobile electronic device, comprising:
a first displacement detection unit that detects a displacement value associated with movement in an up-and-down direction of the mobile electronic device;
a second displacement detection unit that detects a displacement value associated with movement or turning in a predetermined direction of the mobile electronic device;
a walking posture reference locus storage unit that stores a locus serving as a reference in relation to a walking posture;
a characteristic displacement timing extraction unit that extracts a timing of a predetermined characteristic displacement appearing at every step as a walking timing based on upward and downward displacement values detected at a plurality of timings by the first displacement detection unit;
a locus calculation unit that selects successive walking timings extracted by the characteristic displacement timing extraction unit, and calculates a locus caused by walking of the selected walking timings based on the displacement value detected by the second displacement detection unit; and
a walking posture determination unit that determines whether the locus calculated by the locus calculation unit satisfies the reference in relation to a walking posture stored in the walking posture reference locus storage unit.

2. The mobile electronic device according to claim 1, wherein the locus calculation unit comprises:
a right-and-left turning amount calculation unit that calculates, when the second displacement detection unit detects a displacement value associated with turning in a right-and-left direction, a difference between a maximum displacement value caused by an immediately preceding walking timing of the selected walking timing and a maximum displacement value caused by an immediately subsequent walking timing of the selected walking timing as a right-and-left turning locus.

3. The mobile electronic device according to claim 1 or 2, further comprising an output unit that outputs a determination result determined by the walking posture determination unit.

4. A walking locus calculation program for causing a mobile electronic device to execute the procedure, the walking locus calculation program comprising:
extracting a timing of a predetermined characteristic displacement appearing at every step as a walking timing based on upward and downward displacement values detected at a plurality of timings by a first displacement detection unit that detects a displacement value associated with movement in an up-and-down direction of the mobile electronic device;
selecting successive walking timings extracted at the extracting;
calculating a locus caused by walking of the selected walking timings based on a displacement value detected by a second displacement detection unit that detects a displacement value associated with movement or turning in a predetermined direction of the mobile electronic device; and
determining whether the locus calculated at the calculating satisfies a reference in relation to a walking posture stored in a storage unit that stores a locus serving as the reference in relation to the walking posture.

5. A method of diagnosing a walking posture performed by an electronic device, the method comprising:
extracting a timing of a predetermined characteristic displacement appearing at every step as a walking timing based on upward and downward displacement values detected at a plurality of timings by a first displacement detection unit that detects a displacement value associated with movement in an up-and-down direction;
selecting successive walking timings extracted at the extracting;
calculating a locus caused by walking of the selected walking timings based on a displacement value detected by a second displacement detection unit that detects a displacement value associated with movement or turning in a predetermined direction; and
determining whether the locus calculated at the calculating satisfies a reference in relation to a walking posture stored in a storage unit that stores a locus serving as the reference in relation to the walking posture.

6. A mobile electronic device, comprising:
a display unit;
a control unit;
an acceleration sensor; and
an angular velocity sensor that detects angular velocity around an axis perpendicular to an axis along which the acceleration sensor detects acceleration,
wherein the control unit generates
a menu screen including a selection item for performing a form diagnosis,
a diagnosis result screen that displays a diagnosis result including an advice menu based on an output from the acceleration sensor and the acceleration sensor after the form diagnosis is selected, and
an advice screen that displays an advice content after the advice menu is selected, and
the display unit displays
the menu screen, the diagnosis result screen, and the advice screen.
